# EUROPÄISCHE PATENTANMELDUNG

(11) **EP 4 792 798 A2**
(43) Veröffentlichungstag der Anmeldung: **19.08.2026**
(21) Anmeldenummer: 26185309.7
(22) Anmeldetag: 04.10.2024
(51) Int. Cl.: A61J 3/07

(54) **ROBUSTES VERFAHREN ZUR HERSTELLUNG VON WEICHKAPSELN**

(30) Priorität: 06.10.2023 CH 11082023
(62) Dokumentnummer(n) der früheren Anmeldung(en) nach Art. 76 EPÜ: 24799330.6 / 4 788 289
(71) Anmelder: InnoGEL AG, 6331 Hünenberg (CH)
(72) Erfinder: Müller, Rolf, 8046 Zürich (CH); Innerebner, Federico, 8049 Zürich (CH)
(74) Vertreter: IPrime Rentsch Kaelin AG

(57) **Zusammenfassung**

Eine Giessmasse (11) zur Herstellung von auf Stärke basierenden Weichkapseln weist 20 - 45 Gew.-% Wasser auf. Die trockene Giessmasse umfasst 40 - 80 Gew.-% Stärke, wobei mehr als 80 Gew.-% der Stärke als Partikel granulärer nativer Stärke vorliegt; 22 - 65 Gew.-% Weichmacher; 0.5 - 5 Gew.-% Verdickungsmittel. Ein Verfahren zur Herstellung von Weichkapseln mit einem Rotary-Die-Verfahren, umfasst folgende Verfahrensschritte: a) Bereitstellen einer solchen Giessmasse (11); b) Giessen der Giessmasse (11) mit einem Verteilkasten (12) auf eine Giesstrommel (13); c) Umwandlung der Giessmasse (11) auf der Giesstrommel (13) zu einem Band (29) mittels Temperaturerhöhung der Giessmasse (11); d) Abziehen des Bandes (29) von der Giesstrommel (13) und Zuführen des Bandes (29) zu einer Rotary-Die-Verkapselungseinheit (44) bestehend aus Füllkeil (20) und zwei Formwalzen (21); e) Verkapselung eines Füllgutes (19) in der Rotary-Die-Verkapselungseinheit (44), wobei die Weichkapseln gebildet werden; wobei im Schritt d) eine Orientierung der im Band (29) enthaltenen Makromoleküle gering gehalten wird, so dass die Orientierung der Makromoleküle am Ende von Schritt d) in minimalem Umfang vorliegt.

## Beschreibung

### Technisches Gebiet

Die vorliegende Erfindung betrifft ein Verfahren zur Herstellung von vegetarischen Weichkapseln, insbesondere ein Verfahren zur Herstellung von auf Stärke basierenden Weichkapseln, eine Giessmasse zur Herstellung von auf Stärke basierenden Weichkapselhüllen und eine auf Stärke basierende Weichkapsel.

### Technologischer Hintergrund

Seit 1933 werden Weichkapseln auf Basis von Gelatine mit dem Rotary-Die-Verfahren hergestellt. Mit der BSE-Krise in den 90er Jahren entstand der Wunsch nach einer Gelatine-freien, insbesondere vegetarischen, d.h. auf pflanzlichen Rohstoffen basierenden Weichkapsel. In der Folge davon kamen nach 2000 erste vegetarische Weichkapseln auf den Markt, die jedoch nur Nischen besetzen konnten. Im Zuge des generellen Trends zu vegetarischer, veganer, bewusster und gesunder Ernährung wurde das Bedürfnis nach unbedenklichen Weichkapseln immer stärker. Im Jahr 2020 haben die meisten Hersteller von Weichkapseln eine vegane Alternative im Angebot, wobei unter den verschiedenen neuen veganen Weichkapseln solche basierend auf Carrageenan klar am weitesten verbreitet und zu einem neuen Standard geworden sind. Dabei wurde für die Herstellung das altbekannte Rotary-Die-Verfahren beibehalten.

Seit der BSE Krise sind inzwischen bald 30 Jahre vergangen. In dieser Zeit wurden Dutzende vegetarischer bzw. veganer Alternativen entwickelt, von denen es nur sehr wenige bis zur Marktreife gebracht haben. Und von diesen hat nur die Alternative basierend auf Carrageenan einen nennenswerten Marktanteil erreichen können. Schätzungsweise 90% der heutigen veganen Weichkapseln basieren auf Carrageenan. Doch obwohl der Druck hin zu vegetarischen und veganen Lösungen massiv zugenommen hat und in der Zukunft eine weitere starke Zunahme erwartet wird, haben solche Weichkapseln insgesamt immer noch einen nur sehr bescheidenen Marktanteil von bis zur Grössenordnung von etwa 10% erreichen können.

Die Weichkapselhersteller bieten zwar meist auch vegetarische Alternativen an, verkauft werden aber nach wie vor hauptsächlich Gelatine-Weichkapseln, entgegen dem Trend zu vegetarischen und veganen Lösungen, trotz der BSE-Krise, trotz der Fleisch-Skandale, trotz den Komplikationen mit den Koscher- und Halal-Anforderungen die sich bei Gelatine ergeben, entgegen dem Trend zu bewusster Ernährung hinsichtlich Gesundheit und Umwelt. Wobei die Fleischwirtschaft auch hinsichtlich des Wasser- und Energie-Verbrauchs und dem grossen CO₂-Fussabdruck in Verruf geraten ist und hier in Zukunft mit Einschränkungen zu rechnen ist, womit die Verfügbarkeit von Gelatine problematisch werden dürfte.

Dass sich vegetarische Weichkapseln auf dem Markt nicht breit durchsetzen konnten, hat mehrere Gründe. Die Materialkosten einer Carrageenan-basierten Kapselhülle sind mehr als doppelt so gross wie bei der klassischen Gelatine-Kapselhülle. Hinzu kommt noch, dass das Verfahren aufwendiger ist und die Produktionsgeschwindigkeit (Kapseln pro Stunde) reduziert ist. Auf Carrageenan basierende Weichkapseln sind darum deutlich teurer als die herkömmlichen Gelatine-Weichkapseln. Bei den anderen vegetarischen Alternativen sind die Herstellungskosten in einem ähnlich hohen Bereich oder noch höher.

Dann kommen bei der führenden Carrageenan-Alternative noch andere Bedenken hinzu. Im Tierversuch wurden Geschwürbildungen und Veränderungen im Immunsystem im Zusammenhang mit abgebautem Carrageenan festgestellt. Deshalb wird solches Carrageenan im Labor eingesetzt, um beispielsweise bei Ratten chronisch-entzündliche Darmerkrankungen für Forschungszwecke zu generieren. Das für Weichkapseln eingesetzte Carrageenan ist jedoch nicht abgebaut. Doch entsteht Abbau bei hohen Temperaturen und niedrigem pH, und das für Carrageenan Weichkapseln eingesetzte Carrageenan wird bei hohen Temperaturen im Bereich von 90 °C verarbeitet und auch über längere Zeit bei solchen Temperaturen gelagert. Die Lebensmittelverordnungen erlauben den Einsatz von Carrageenan unter Einschränkungen. Es ist jedoch möglich, dass es hier in der Zukunft zu grösseren Einschränkungen kommt, wenn neue Forschungsresultate vorliegen.

Insgesamt ist die Situation hinsichtlich einer veganen und völlig unbedenklichen alternativen Weichkapsel nach bald 30 Jahren immer noch alles andere als befriedigend, während der Druck für eine solche Lösung weiter zunimmt.

WO 2010/100206 A1 und WO 2010/100196 A1 der Anmelderin beschreiben ein Verfahren und eine Vorrichtung zur Herstellung von Stärkefolien und daraus hergestellte Weichkapseln auf Stärkebasis, wobei hierzu eine viskose Mischung aus Stärke, Wasser und Weichmacher verwendet wird, in welcher mehr als 50% des Gewichts der Stärke in Form von nicht destrukturierten, doppelbrechenden Partikeln vorliegt. In einem Formgebungsprozess wird die Mischung zu einem Film umgeformt, woraufhin die viskose Mischung durch ein Erhöhen der Temperatur verfestigt wird, indem die Stärke-Partikel destrukturiert werden. Der Film weist dann miteinander verbundene Stärke-Partikel auf. In einem nächsten Schritt wird dieser Film mit dem Rotary-Die-Verfahren zu Weichkapseln umgeformt, wobei die Weichkapseln geformt, gefüllt und entformt werden.

Dieses Verfahren ist bei hohen Produktionsgeschwindigkeiten empfindlich gegenüber Änderungen aus der Umgebung und gegenüber Änderungen der Verfahrensparameter. Die Qualität der Weichkapseln nimmt mit zunehmender Produktionsgeschwindigkeit ab, weshalb hier eine Limitierung der Drehzahl auf rund 3.5 U/min bestand.

Bei den Herstellungsverfahren aus dem Stand der Technik nehmen die Robustheit des Prozesses und die Qualität der Produkte mit zunehmender Produktionsgeschwindigkeit ab, womit die Wirtschaftlichkeit deutlich beeinträchtigt wird. Dies ist generell bei allen Verfahren zur Herstellung von Weichkapseln der Fall. Insbesondere bei Verfahren zur Herstellung von vegetarischen Weichkapseln ist dies ein charakteristisches Verhalten, was zusätzlich zu den höheren Rohstoffkosten mit den gegenüber dem Gelatine-Verfahren tiefen Produktionsgeschwindigkeiten zu höheren Herstellungskosten führt. Diese Kostendifferenz ist ein wichtiger Grund, weshalb Gelatine-Weichkapseln trotz dem starken Trend hin zu vegetarischer und veganer Ernährung immer noch den Markt dominieren.

Aus dem Stand der Technik ist WO 2007/128150 A1 der hiesigen Anmelderin bekannt. Diese Druckschrift betrifft ein sog. «Tieftemperatur-Mogul-Verfahren», mit welchem Konfektartikel, d.h. Süsswaren auf Basis von Stärke herstellbar sind. Ein ausschlaggebendes Merkmal dieses Verfahrens ist, dass die Giessmasse aus Stärke bei vergleichsweise niedriger Temperatur gegossen wird und die Gelierung bzw. Lagerung bei vergleichsweise hoher Temperatur stattfindet. In dem Verfahren wird zumindest ein Anteil der Stärke erst nach dem Giessen in eine Form vollständig aufgelöst. Die Umwandlung der Giessmasse in einen festen Stoff dauert bei diesem Verfahren rund 3 Tage, während beim Rotary-Die-Verfahren die Umwandlung der Giessmasse in ein festes Band im Bereich von Sekunden stattfinden muss. Daher ist das Tieftemperatur-Mogul-Verfahren, das für die Herstellung von Süsswaren entwickelt wurde und eingesetzt wird, nicht für Weichkapseln adaptierbar, wo ausserdem auch ganz andere Anforderungen gelten.

Die Marktkräfte waren gross genug, dass auf Carrageenan basierende Weichkapseln trotz der Bedenken zu diesem Stoff und trotz den massiv höheren Kosten im Vergleich zu Gelatine immerhin einen Marktanteil von bis zur Grössenordnung von 10% erreichen konnte. Wenn nun der Vorteil der pflanzlichen Basis mit gesundheitlicher Unbedenklichkeit und tieferen Herstellungskosten im Vergleich zur Gelatine-Weichkapsel kombinierbar wäre, könnte Gelatine nach bald 100-jähriger Dominanz substanziell im Markt für Weichkapseln ersetzt werden.

Es besteht somit ein allgemeines Bedürfnis nach Verbesserungen in diesem Gebiet.

### Darstellung der Erfindung

Eine Aufgabe der Erfindung besteht darin, ein Verfahren zur Herstellung von vegetarischen, insbesondere veganen, Weichkapseln, insbesondere ein Verfahren zur Herstellung von auf Stärke basierenden Weichkapseln, zur Verfügung zu stellen, welches mindestens einem der oben erwähnten und anderer Nachteile entgegenwirkt.

Insbesondere ist es die Aufgabe der Erfindung, die Technologie zur Herstellung von Stärke-Weichkapseln hinsichtlich Robustheit, Qualität der Produkte und Produktionsgeschwindigkeit entscheidend zu verbessern, sodass die verbesserte Technologie der traditionellen Gelatine-Technologie hinsichtlich dieser Aspekte mindestens vergleichbar ist, daneben aber wegen der deutlich geringeren Rohstoffkosten der Kapselhülle wirtschaftlich relevant überlegen ist.

Die Technologie zur Herstellung von Stärke-Weichkapseln nutzt die Phasenumwandlung der Gelatinisierung von Stärke, welche unter Erhitzen stattfindet und wodurch eine flüssige Masse in einen mehr oder weniger festen Stoff bzw. einen viskoelastischen Stoff umgewandelt wird. Diese Technologie unterscheidet sich grundsätzlich von anderen Technologien zur Herstellung von Weichkapseln, wo die Verfestigung der flüssigen Giessmasse durch eine Kühlung derselben erhalten wird und wobei insbesondere bei der Kühlung ein Geliermittel wie beispielsweise Gelatine, Carrageenan, Gellan oder Pektin durch Gelbildung zu dieser Verfestigung führt.

Mit dem erfindungsgemässen Herstellungsverfahren sollen vegetarische Weichkapseln wie die traditionellen Gelatine-Weichkapseln mit dem Rotary-Die-Verfahren produzierbar sein. Die hergestellten Weichkapseln sollen eine Textur erhalten, welche der typischen Textur von Gelatine ähnlich ist, ohne dass hierzu notwendigerweise Gelatine eingesetzt werden muss.

Die Erfindung beschreibt ein flexibles, robustes und effizientes Verfahren zur Herstellung von vegetarischen, insbesondere veganen, Weichkapseln, insbesondere von Weichkapseln auf Basis von Stärke.

Da die Rohstoffkosten der auf Stärke basierten Kapselhülle nur etwa 30% der Rohstoffkosten der auf Gelatine basierten Weichkapsel betragen, kommen zum Vorteil der vegetarischen und veganen Eigenschaft noch massive Kosteneinsparungen hinzu. Gegenüber anderen pflanzlichen Alternativen sind die Kosteneinsparungen noch signifikant höher, die Rohstoffkosten liegen hier im Vergleich bei nur etwa 15%.

Mit dem neuen Verfahren können gegenüber der Produktion von herkömmlichen vegetarischen Weichkapseln höhere Produktionsgeschwindigkeiten erreicht werden und es sind sogar deutlich höhere Produktionsgeschwindigkeiten möglich. Da die neue Weichkapsel mit natürlichen Rohstoffen hergestellt werden kann, ist einmalig eine Clean Label Qualität möglich und sogar Bio-Qualität. Damit kann die neue Weichkapsel die aus Schlachtabfällen gewonnene Gelatine im Weichkapselbereich überflüssig machen, was mit den bisherigen Alternativen nicht gelungen ist.

Diese und andere Aufgaben werden gelöst durch ein erfindungsgemässes Verfahren zur Herstellung von Weichkapseln, eine erfindungsgemässen Giessmasse zur Herstellung von auf Stärke basierenden Weichkapselhüllen, sowie eine durch eine erfindungsgemässe auf Stärke basierende Weichkapsel, gemäss den unabhängigen Patentansprüchen. Weitere vorteilhafte Ausführungsformen gehen ausserdem aus den abhängigen Ansprüchen und der Beschreibung hervor.

Die erfindungsgemässe Lösung kann durch verschiedene, jeweils für sich vorteilhafte und, sofern nicht anders ausgeführt, miteinander kombinierbare Ausgestaltungen weiter verbessert werden. Auf diese Ausführungsformen und die mit ihnen verbundenen Vorteile ist im Folgenden eingegangen.

Ein Aspekt der Erfindung betrifft ein Verfahren zur Herstellung von Weichkapseln mit einem Rotary-Die-Verfahren.

Ein erfindungsgemässes Verfahren umfasst folgende Verfahrensschritte:
a) Bereitstellen einer Giessmasse;
b) Giessen der Giessmasse mit einem Verteilkasten auf eine Giesstrommel;
c) Umwandlung der Giessmasse auf der Giesstrommel zu einem Band mittels Temperaturerhöhung der Giessmasse;
d) Abziehen des Bandes von der Giesstrommel und Zuführen des Bandes zu einer Rotary-Die-Verkapselungseinheit bestehend aus Füllkeil und zwei Formwalzen;
e) Verkapselung eines Füllgutes in der Rotary-Die-Verkapselungseinheit, wobei die Weichkapseln gebildet werden;
wobei im Schritt d) eine Orientierung der im Band enthaltenen Makromoleküle gering gehalten wird, so dass die Orientierung der Makromoleküle am Ende von Schritt d) in minimalem Umfang vorliegt.

In Schritt d) wird vorteilhaft zwischen dem Abziehen des Bandes von der Giesstrommel und vor dem Zuführen des Bandes zur Rotary-Die-Verkapselungseinheit das Band gesamt oder bereichsweise beölt.

Nach einer optionalen Vorkonditionierung und Konditionierung können die fertigen Weichkapseln sortiert und verpackt werden.

In einer vorteilhaften Variante des erfindungsgemässen Verfahrens wird in Schritt d) eine Verstreckung S des Bandes gering gehalten, insbesondere kleiner als 20%, wobei die Verstreckung S in Prozent aus der Umfanggeschwindigkeit V1 der Giesstrommel und der Umfanggeschwindigkeit V2 der Formwalze der Rotary-Die-Verkapselungseinheit mit S = 100*(V2-V1)/V1 erhalten wird.

Die Verstreckung S ist dabei als eine relative Streckung des Bandes in Förderrichtung zu verstehen.

Vorteilhaft wird in einem erfindungsgemässen Verfahren die Giesstrommel während des Verfahrens gereinigt, vorteilhaft kontinuierlich gereinigt.

Alternativ oder zusätzlich kann in einem erfindungsgemässen Verfahren die Giesstrommel mit einer die Haftung des Bandes auf der Giesstrommel reduzierenden Schicht beschichtet sein.

In einer anderen vorteilhaften Variante des erfindungsgemässen Verfahrens wird auf die Oberfläche des Bandes, welche schliesslich die Aussenseite der Weichkapselhülle bildet in Schritt d) im Verfahrensbereich ab Giesstrommel bis Formwalze, über eine Aussenbeölung Öl in einer Menge von höchstens 2 g/m² aufgetragen.

In einer weiteren vorteilhaften Variante des erfindungsgemässen Verfahrens wird auf die Oberfläche des Bandes, welche schliesslich die Aussenseite der Weichkapselhülle bildet, in Schritt d) im Verfahrensbereich ab Giesstrommel bis Formwalze, kein Öl aufgetragen.

In einem erfindungsgemässen Verfahren kann nach der Verkapselung zusätzlich oder alternativ eine Aussenbeölung stattfinden.

Eine solche Aussenbeölung findet vorteilhaft im Förderkanal statt, mit dem die Weichkapseln einer nachfolgenden Vorkonditionierung zugeführt werden.

In einer vorteilhaften Variante des erfindungsgemässen Verfahrens werden die fertigen Weichkapseln nicht entölt.

In einer anderen vorteilhaften Variante des erfindungsgemässen Verfahrens liegt die Temperatur des Bandes unmittelbar vor dem Einzug unter den Füllkeil der Rotary-Die-Verkapselungseinheit im Bereich von 15 bis 60 °C.

Die Temperatur des Bandes wird mit einem IR-Thermometer bestimmt.

Vorteilhaft wird in einem erfindungsgemässen Verfahren das Band im Schritt d) aktiv gekühlt.

Die aktive Kühlung kann beispielsweise durch einen Luftstrom und/oder durch mindestens eine gekühlte Walze erfolgen.

Alternativ oder zusätzlich kann eine vorteilhafte Temperatur des Bandes durch eine entsprechend lange Verfahrenslänge im Schritt d) des Verfahrens erreicht werden.

Vorteilhaft weist in einem erfindungsgemässen Verfahren der Füllkeil der Rotary-Die-Verkapselungseinheit im Innern eine Temperatur im Bereich von 10 bis 60 °C auf.

Die Temperatur des Füllkeiles wird mit einem Thermoelement bestimmt.

Vorteilhaft wird der Füllkeil aktiv gekühlt.

Vorteilhaft weisen in einem erfindungsgemässen Verfahren die Formwalzen der Rotary-Die-Verkapselungseinheit auf ihrer Oberfläche eine Temperatur im Bereich von 0 bis 45 °C auf.

Vorteilhaft werden die Formwalzen aktiv gekühlt, beispielsweise durch einen Luftstrom und/oder durch eine Kühlung mit einem flüssigen Medium.

Die Temperatur der Oberfläche der Formwalzen wird mit einem IR-Thermometer bestimmt.

Vorteilhaft weisen in einem erfindungsgemässen Verfahren die Hüllen der Weichkapseln unmittelbar nach der Verkapselung eine Temperatur von nicht mehr als 50 °C auf.

Die Temperatur der Hüllen der Weichkapseln wird mit einem IR-Thermometer bestimmt.

In einer vorteilhaften Variante des erfindungsgemässen Verfahrens werden die frisch hergestellten Weichkapseln nach der Verkapselung aktiv gekühlt.

Eine solche aktive Kühlung kann beispielsweise in einem Förderkanal erfolgen.

In einer weiteren vorteilhaften Variante des erfindungsgemässen Verfahrens werden die frisch hergestellten Weichkapseln einer Vorkonditionierung zugeführt und dabei gekühlt.

Eine solche Vorkonditionierung kann mit einer Tumblervorrichtung und/oder einem Fluidbett durchgeführt werden.

Vorteilhaft weisen in einem erfindungsgemässen Verfahren die Weichkapseln nach der Vorkonditionierung an der Hülle wie auch im Innern eine Temperatur von nicht mehr als 35 °C auf.

Die Temperatur der Hüllen der Weichkapseln wird wiederum mit einem IR-Thermometer bestimmt.

Die Temperatur des Füllguts im Innern der Weichkapseln wird mit einem drahtförmigen Thermoelement bestimmt, wobei der Draht mit der Messstelle durch die Hülle ins Füllgut gesteckt wird.

In einer anderen vorteilhaften Variante des erfindungsgemässen Verfahrens werden die Weichkapseln einer Konditionierung (7) zugeführt und dabei getrocknet.

Besonders vorteilhaft sind die Weichkapseln nach der Konditionierung bei Raumtemperatur im Gleichgewicht mit einer Luftfeuchtigkeit von 13 bis 40%, vorteilhaft von 15 bis 37%, noch vorteilhafter von 17 bis 35%, und noch vorteilhafter von 19 bis 33%.

Ein Gleichgewicht mit der Luftfeuchtigkeit ist dabei so zu verstehen, dass bei einer bestimmten Luftfeuchtigkeit sich der Wassergehalt der Hülle der Weichkapsel nicht mehr ändert. Es liegt ein Gleichgewicht vor.

In noch einer weiteren vorteilhaften Variante des erfindungsgemässen Verfahrens drehen die Formwalzen der Rotary-Die-Verkapselungseinheit mit einer Umfanggeschwindigkeit V2, die bei einem Durchmesser der Formwalzen von 150 mm einer Drehgeschwindigkeit von mehr als 3.5 U/min entspricht, vorteilhaft von mehr als 4 U/min, noch vorteilhafter von mehr als 4.5 U/min, und noch vorteilhafter von mehr als 5 U/min.

Vorteilhaft wird in einem erfindungsgemässen Verfahren das Band aus einer granuläre Stärke aufweisenden Giessmasse erhalten, die auf einer geheizten Giesstrommel durch eine Temperaturerhöhung, die zur Gelatinisierung der granulären Stärke führt, in ein viskoelastisches Material umgewandelt wird.

Besonders vorteilhaft ist in einem erfindungsgemässen Verfahren die Weichkapsel eine vegetarische oder vegane Weichkapsel, insbesondere eine Stärke-Weichkapsel.

Vorteilhaft weist in einem erfindungsgemässen Verfahren das auf der Giesstrommel geformte Band eine Mikrostruktur auf, wobei gelatinisierte Stärke-Körner aneinander liegen und miteinander verbunden sind.

In einem erfindungsgemässen Verfahren weist vorteilhaft die Giessmasse 20 - 45 Gew.- % Wasser auf und die trockene Giessmasse umfasst vorteilhaft folgende Komponenten:
40 - 80 Gew.-% Stärke, wobei mehr als 80 Gew.-% der Stärke als Partikel granulärer Stärke vorliegt;
20 - 65 Gew.-% Weichmacher;
0 - 7 Gew.-% Verdickungsmittel.

Unter dem Begriff «trockene Giessmasse» ist dabei im Rahmen dieser Beschreibung die Giessmasse ohne den Wasseranteil gemeint. Das heisst, die entsprechenden Anteile beziehen sich auf die Giessmasse nach der rechnerischen Entfernung des Wasseranteils.

Unter dem Wasser wird hier das totale Wasser verstanden, dass sich aus dem zugegebenen Wasser und dem in den Komponenten als Feuchtigkeit enthaltenen Wasser ergibt. Die Anteile der Komponenten beziehen sich entsprechend auf die wasserfreien Komponenten.

In einer vorteilhaften Variante des Verfahrens umfasst die trockene Giessmasse 0 - 7 Gew.-% gelöste Stärke.

Das Verdickungsmittel verlangsamt insbesondere die Sedimentation der Stärkekörner und verlängert so die Lagerzeit der Giessmasse. Der Fachmann erkennt, dass das vollständige Weglassen des Verdickungsmittel (0 Gew.-%) nur in besonderen Fällen vorgesehen würde, dass aber grundsätzlich mindestens ein geringer Anteil von Verdickungsmittel vorteilhaft ist.

Vorteilhaft enthält das Verdickungsmittel mindestens eine Komponente aus folgender Liste: Guarkernmehl, Johannisbrotkernmehl, Xanthan.

Besonders vorteilhaft enthält in einer erfindungsgemässen Verfahren das Verdickungsmittel im Wesentlichen Xanthan.

Ebenfalls vorteilhaft liegt der Anteil des Verdickungsmittels im Bereich von 0,5 bis 5 Gew.-% der trockenen Giessmasse.

Gegebenenfalls kann die Giessmasse übliche Zusatzstoffe und Hilfsstoffe enthalten.

Ein weiterer Aspekt der Erfindung betrifft eine vegetarische insbesondere vegane, auf Stärke basierende Weichkapsel.

Eine erfindungsgemässe Stärke-Weichkapsel ist nach einem erfindungsgemässen Verfahren hergestellt.

Vorteilhaft weist bei einer erfindungsgemässen Stärke-Weichkapsel die Hülle der Weichkapsel Körner von gelatinisierter Stärke auf.

Gemäss dem Wiedergewinnungsverfahren Nr. 2 können aus der Weichkapselhülle Körner von gelatinisierter Stärke zu einem Anteil von mindestens 25%, bezogen auf das Gewicht der trockenen Weichkapselhüllen-Masse, wiedergewonnen werden.

Ein weiterer Aspekt der Erfindung betrifft eine Giessmasse zur Herstellung von auf Stärke basierenden Weichkapselhüllen.

Eine erfindungsgemässe Giessmasse weist 20 - 45 Gew.-% Wasser auf und die trockene erfindungsgemässe Giessmasse umfasst folgende Komponenten:
40 - 80 Gew.-% Stärke, wobei mehr als 80 Gew.-% der Stärke als Partikel granulärer Stärke vorliegt;
20 - 65 Gew.-% Weichmacher;
0 - 7 Gew.-% Verdickungsmittel.

Unter dem Begriff «trockene Giessmasse» ist dabei im Rahmen dieser Beschreibung die Giessmasse ohne den Wasseranteil gemeint. Das heisst, die entsprechenden Anteile beziehen sich auf die Giessmasse nach der rechnerischen Entfernung des Wasseranteils.

Unter dem Wasser wird hier das totale Wasser verstanden, dass sich aus dem zugegebenen Wasser und dem in den Komponenten als Feuchtigkeit enthaltenen Wasser ergibt. Die Anteile der Komponenten beziehen sich entsprechend auf die wasserfreien Komponenten.

In einer vorteilhaften Variante umfasst die trockene Giessmasse 0 - 7 Gew.-% gelöste Stärke.

Das Verdickungsmittel und/oder die gelöste Stärke verlangsamen insbesondere die Sedimentation der Stärkekörner und verlängern so die Lagerzeit der Giessmasse. Der Fachmann erkennt, dass das vollständige Weglassen des Verdickungsmittel (0 Gew.- %) nur in besonderen Fällen vorgesehen würde, dass aber grundsätzlich mindestens ein geringer Anteil von Verdickungsmittel vorteilhaft ist.

Vorteilhaft enthält in einer erfindungsgemässen Giessmasse das Verdickungsmittel mindestens eine Komponente aus folgender Liste: Guarkernmehl, Johannisbrotkernmehl, Xanthan.

Besonders vorteilhaft enthält in einer erfindungsgemässen Giessmasse das Verdickungsmittel im Wesentlichen Xanthan.

Besonders vorteilhaft liegt der Anteil des Verdickungsmittels in der Giessmasse im Bereich von 0,5 bis 5 Gew.-% der Mischung.

Gegebenenfalls kann die Giessmasse übliche Zusatzstoffe und Hilfsstoffe enthalten.

Vorteilhaft kann aus der erfindungsgemässen Giessmasse durch Gelatinisieren der in der Giessmasse enthaltenen granulären Stärke ein Film erhalten werden, der einen E-Modul von mindestens 0,55 MPa aufweist.

Eine solche erfindungsgemässe Giessmasse wird besonders vorteilhaft zur Herstellung von Stärke-Weichkapseln eingesetzt.

Ein weiterer Aspekt der Erfindung betrifft eine Stärke-Weichkapsel.

Bei einer erfindungsgemässen Stärke-Weichkapsel umfasst die wasserfreie Weichkapselhülle folgende Komponenten:
40 - 80 Gew.-% Stärke;
20 - 65 Gew.-% Weichmacher;
0 - 7 Gew.-% Verdickungsmittel.

Unter dem Begriff «wasserfreie Weichkapselhülle» ist dabei im Rahmen dieser Beschreibung die Weichkapselhülle nach vollständiger Entfernung des nach abgeschlossener Herstellung verbleibenden Wasseranteils in der Weichkapselhülle gemeint. Zur Analyse der Inhaltsstoffe wird die Weichkapselhülle somit vollständig getrocknet. Alternativ kann der Wasseranteil weggerechnet werden.

Vorteilhaft umfasst die wasserfreie Weichkapselhülle 0 - 7 Gew.-% gelöste Stärke.

Gegebenenfalls kann die Weichkapselhülle übliche Zusatzstoffe und Hilfsstoffe enthalten.

Vorteilhaft können bei einer erfindungsgemässen Stärke-Weichkapsel mit dem Wiedergewinnungsverfahren Nr. 2 aus der Hülle der Weichkapsel mindestens 25 Gew.-% gelatinisierte Stärkekörner zurückgewonnen werden, bezogen auf das Gewicht der wasserfreien Weichkapsel-Hülle.

Vorteilhaft weist in einer erfindungsgemässe Weichkapselhülle das Verdickungsmittel mindestens eine Komponente aus folgender Liste: Guarkernmehl, Johannisbrotkernmehl, Xanthan.

Besonders vorteilhaft enthält in einer erfindungsgemässen Weichkapselhülle das Verdickungsmittel im Wesentlichen Xanthan.

Besonders vorteilhaft liegt der Anteil des Verdickungsmittels in der wasserfreien Weichkapsel-Hülle im Bereich von 0,5 bis 5 Gew.-%.

Wenn vorliegend auf ein festes Band bzw. ein festes, elastisches Band verwiesen wird, ist dabei im Rahmen der Erfindung angesprochen, dass dieses im Rahmen des Verfahrens eine hinreichende Stabilität besitzt. Das Band ist aber formbar und bis zu einem gewissen Grade verstreckbar. Durch die geeignete Führung des erfindungsgemässen Verfahrens werden diese Eigenschaften gezielt und in gewünschtem Masse beeinflusst und (gesteuert/geregelt) kontrolliert.

Bei einer Stärke-Weichkapsel gemäss der Erfindung kann vorteilhafterweise der Anteil des Verdickungsmittels in der wasserfreien Weichkapsel-Hülle im Bereich von 0,25 bis 3 Gew.-% liegen.

Erfindungsgemässe Stärke-Weichkapseln können vorteilhaft aus einer in Rahmen der Erfindung offenbarten Giessmasse hergestellt werden.

Weitere Aspekte der vorliegenden Erfindung ergeben sich auch aus der nachfolgenden Beschreibung.

In besonderen Ausführungsformen sind in der Beschreibung aufgeführte vorteilhafte Ausführungen zu verschiedenen Merkmalen oder Aspekten des Verfahrens, einzeln oder in Kombination besonders vorteilhaft.

### Kurze Beschreibung der Zeichnungen

Zum besseren Verständnis der vorliegenden Erfindung wird nachfolgend auf die Zeichnungen Bezug genommen. Diese zeigen lediglich Ausführungsbeispiele des Erfindungsgegenstands. Für gleiche oder gleich wirkende Teile werden in den nachfolgenden Figuren und der dazugehörigen Beschreibung gleiche oder ähnliche Bezugszeichen verwendet.
- Figur 1: zeigt schematisch die Verfahrensschritte eines erfindungsgemässen Herstellungsverfahrens.
- Figur 2: zeigt schematisch im Detail eine Rotary-Die-Vorrichtung, wie sie in dem Verfahren nach Figur 1 eingesetzt werden kann.
- Figur 3: zeigt lichtmikroskopische Aufnahmen unter gekreuzten Polarisatoren von (a) einer wässrigen Suspension von unverarbeiteter, nativer, granulärer Tapioka-Stärke, wobei die kleinen Körner eine Grösse im Bereich von einigen µm, die grösseren von bis etwa 15 µm haben; und (b) einer wässrigen Suspension derselben Tapioka- Stärke, nachdem sie in einer erfindungsgemässen Giessmasse zur Herstellung eines Bandes für Weichkapselhüllen auf rund 100 °C erhitzt worden ist. Die Breite des Bildes entspricht in beiden Fällen 570 µm.
- Figuren 4 und 4a: zeigen bei einer Anlage zur Durchführung des erfindungsgemässen Verfahrens die Weiterführung des zuvor gegossenen und hier verfestigten Bandes zur Verkapselungseinheit (auf dem Bild nicht dargestellt).
- Figuren 5 und 5a: zeigen bei einer Anlage zur Durchführung des erfindungsgemässen Verfahrens die Zuführung des rechten Bandes zur rechten Seite der Verkapselungseinheit bestehend aus Füllkeil (wedge) und Formwalzen (die rolls). Auf der linken Seite geschieht mit dem linken Band in symmetrischer Anordnung dasselbe, ist in dem Bild jedoch nicht sichtbar.
- Figur 6: zeigt schematisch eine vorteilhafte Kühlvorrichtung zur Kühlung des Bands.
- Figur 7: zeigt eine Ablöserolle 39 gemäss einer besonderen Ausführungsform der Erfindung.

### Ausführung der Erfindung

Die im Folgenden gegebenen Beispiele dienen der besseren Veranschaulichung der Erfindung, sind jedoch nicht dazu geeignet, die Erfindung auf die hierin offenbarten Merkmale zu beschränken.

In der nachfolgenden Beschreibung sind gleiche oder gleich wirkende Teile mit den gleichen oder ähnlichen Bezugszeichen versehen.

### Herstellung von Weichkapseln

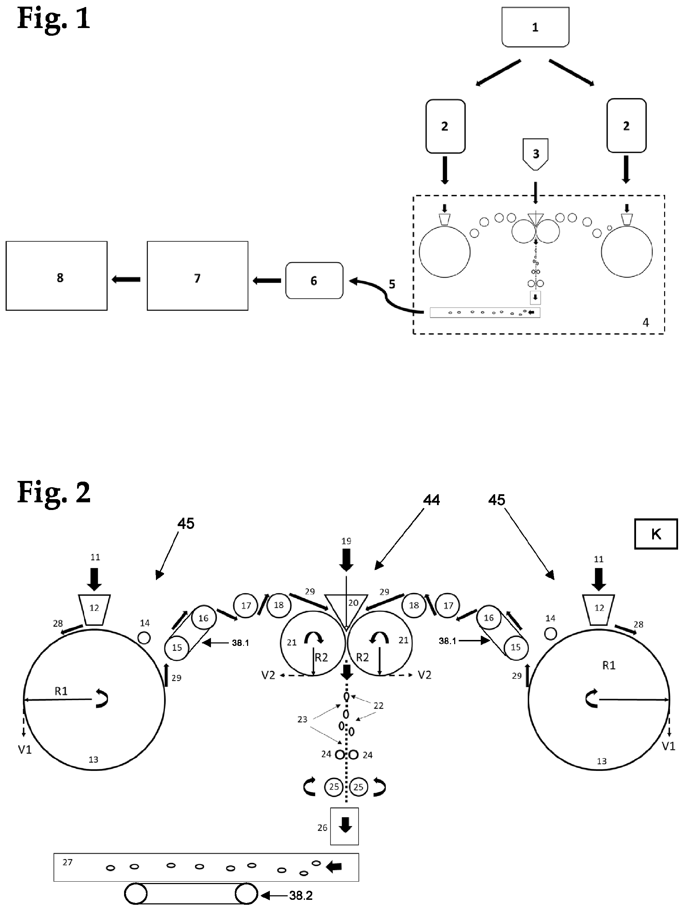

Die grundsätzlichen Verfahrensschritte zur Herstellung von Weichkapseln sind in Figur 1 gezeigt.

Die zentrale Rotary-Die-Vorrichtung 4, mit der das Füllgut 19 in die Weichkapseln verkapselt wird, ist bezüglich der wichtigsten Elemente in Figur 2 schematisch dargestellt.

In einem ersten Schritt wird in einem Mischer 1 eine viskose Giessmasse (casting mass) 11 hergestellt, die in homogener Form, mit einer bestimmten Temperatur und ohne Luftblasen vorliegen soll. Daher beinhaltet die Herstellung der Giessmasse 11 neben dem Mischprozess der Bestandteile auch eine Erwärmung und Entgasung der Giessmasse.

Die fertige Giessmasse 11 wird in Vorratstanks (storage tanks) 2 transferiert, wo die Giessmasse 11 auf einer gewünschten Temperatur gehalten werden kann und von wo die Giessmasse 11 kontinuierlich zur Rotary-Die-Vorrichtung 4 gepumpt wird. In der Regel werden die zwei Verteilkästen (spreader boxes) 12 der Rotary-Die-Vorrichtung 4 von zwei separaten Vorratstanks 2 kontinuierlich mit Giessmasse 11 versorgt. Dies kann nützlich sein, wenn die beiden Weichkapselhälften verschieden eingefärbt sein sollen.

Es ist auch möglich, dass die Verteilkästen 12 mit nur einem Vorratstank 2 versorgt werden.

In der Rotary-Die-Vorrichtung 4 werden aus der Giessmasse 11 zwei Bänder (ribbon) 29 hergestellt, aus denen in der Mitte der Vorrichtung in der Rotary-Die-Verkapselungseinheit 44 die beiden Hüllen-Hälften der Weichkapseln entstehen, welche das verkapselte Füllgut 19 umschliessen und welche über eine rund um die Weichkapsel laufende Naht fest miteinander verbunden sind.

Die frischen Weichkapseln 22 werden mit einem Förderband zu einem Förderkanal 5 transportiert, in welchem die Weichkapseln mit einem Luftstrom einer Vorkonditionierung 6 zugeführt werden.

In einem vergleichsweise kurzen Vorkonditionierungsschritt 6 werden die Weichkapseln gegebenenfalls gekühlt und/oder entölt.

Anschliessend an die Vorkonditionierung 6 wird die Konditionierung 7 der Weichkapseln durchgeführt. Im genannten Konditionierungsschritt 7 werden die Weichkapseln in einem klimatisierten Trocknungsraum während einer bestimmten Zeitdauer im Bereich von 24 h oder mehr konditioniert, und insbesondere auf den gewünschten Endwassergehalt getrocknet, wobei aus der noch sehr weichen, frischen Weichkapsel 22 die fertige, vergleichsweise harte Weichkapsel entsteht.

In einem anschliessenden Schritt 8 wird eine Qualitätskontrolle und Sortierung durchgeführt und die Weichkapseln werden verpackt, beispielsweise in Blister eingeschweisst oder in grösseren Mengen vorverpackt.

### Rotary-Die-Verkapselung

Die für das Verfahren zentrale Rotary-Die-Vorrichtung 4 ist in Figur 2 schematisch dargestellt.

Die Vorrichtung besteht aus einer im Wesentlichen spiegelsymmetrischen Anordnung der Komponenten. Links und rechts wird in zwei Verteilkästen 12 in gleicher Art und Weise kontinuierlich aus der flüssigen, viskosen Giessmasse 11 ein mehr oder weniger festes, elastisches Band 29 hergestellt.

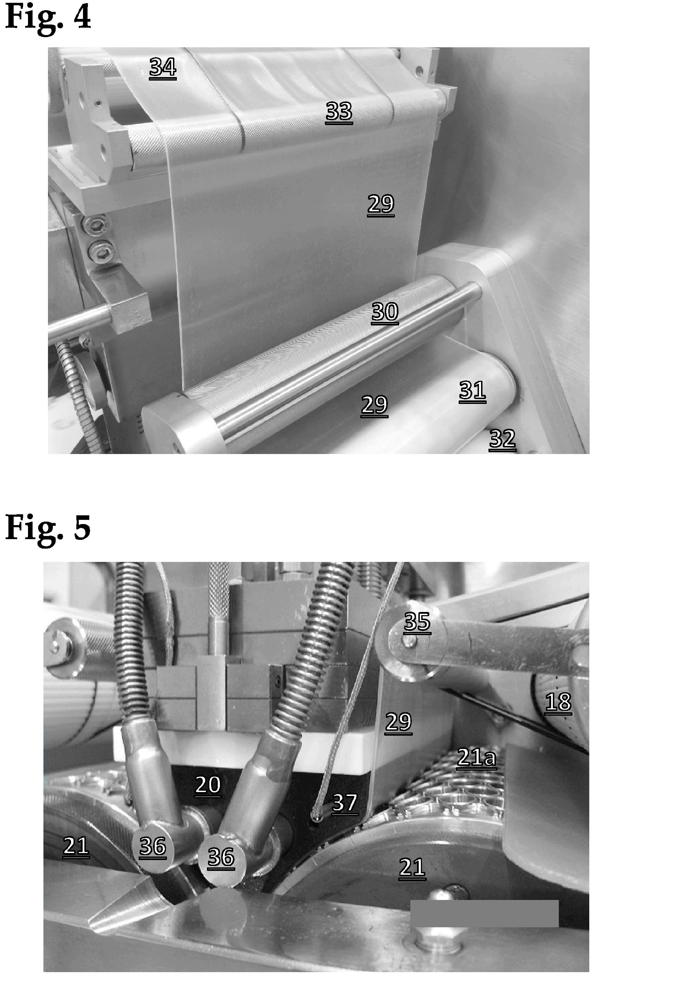

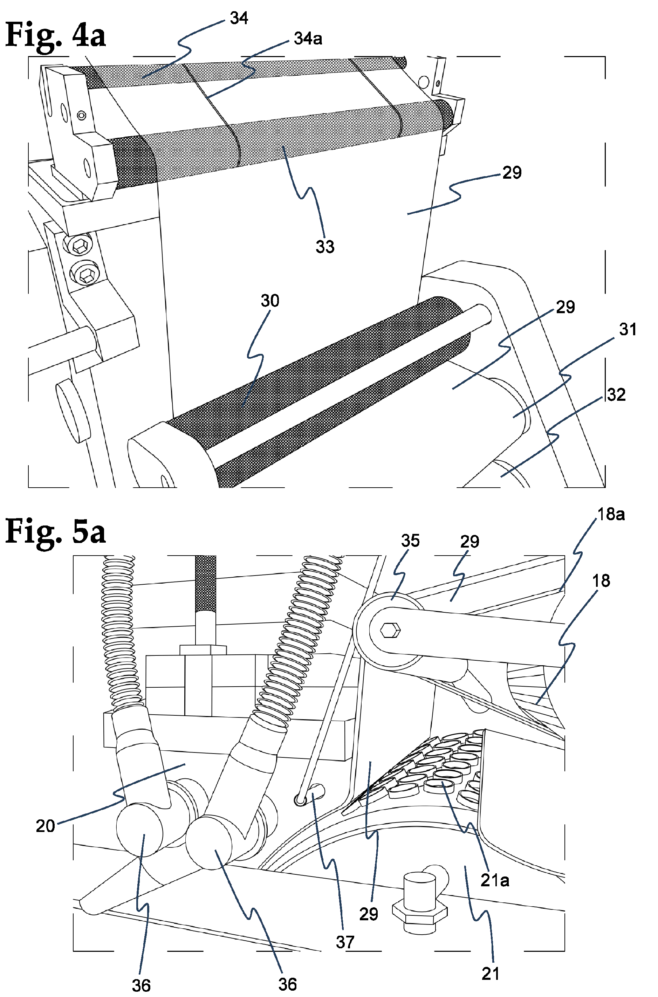

Beispielhaft sind Teile der Vorrichtung auch in Bildern einer beispielhaften Vorrichtung in den Figuren 4, 4a und 5, 5a gezeigt.

Die Giesseinheiten 45 umfassen jeweils ein Verteilkasten 12 und eine Giesstrommel 13. Die Verteilkästen 12 weisen an einem unteren Ende einen Giessspalt auf, durch den die Giessmasse 11 kontinuierlich auf die sich drehenden Giesstrommeln 13 gegossen werden kann.

Aus den temperierten Verteilkästen 12 fliesst die Giessmasse 11 unter Eigengewicht oder unter Druck, der gezielt appliziert wird (sog. «pressurized spreader boxes»), durch den Giessspalt auf die sich kontinuierlich drehende Giesstrommel 13. Die Giesstrommel 13 hat einen Radius R1 und weist eine über die Drehzahl einstellbare Oberflächengeschwindigkeit V1 auf.

Auf der Oberfläche der Giesstrommel 13 entsteht aus der Giessmasse 11 kontinuierlich ein zunächst noch flüssiger Film 28, der dann fest wird. Es resultiert ein festes Band 29.

Bezüglich der für die Gelatinisierung der Stärke in der Giessmasse 11 auf den geheizten Giesstrommeln 13 notwendigen Massnahmen, wobei insbesondere der gegossene Stärke-Film mit einem mitumlaufenden Abdeckband abgedeckt wird (in Figur 2 nicht dargestellt), wird auf die in WO 2010/100196 A1 beschriebene Vorrichtung und das entsprechende Verfahren verwiesen.

Nach etwa einer 270°-Umdrehung wird das Band 29 von der Giesstrommel 13 über die Abzugswalze 15 abgezogen.

Die Giesstrommel 13 wird über eine Polierwalze 14 kontinuierlich gereinigt. Diese neue Massnahme ist im Hinblick auf die Homogenität und die Minimierung der Verstreckung des Bandes 29 von besonderem Vorteil.

Anschliessend wird das noch heisse Band 29, langsam passiv abgekühlt, während es über Walzen 16, 17, 18 bis zur zentralen Rotary-Die-Verkapselungseinheit bestehend aus Füllkeil 20 und Formwalzen 21 geführt wird. Damit das Band 29 straff gehalten werden kann, und so einen glatten Einzug unter den Füllkeil 20 zu erhalten, ist das Band zwischen Giesstrommel 13 und Formwalzen 21 verstreckt. Das heisst, das Band wird in Förderrichtung gedehnt.

Zu Anzahl, Grösse und genauer Positionierung sowie Ausführung dieser Walzen gibt es verschiedene mögliche Ausführungsformen.

Eine wichtige Aufgabe der Verfahrensstrecke von der Abzugswalze 15 bis zum Füllkeil 20 ist die beidseitige Beölung des Bandes 29.

Die Innenseite des Bandes 29, welche bei der fertigen Kapsel die Innenseite bildet, wird über die Innenbeölung beölt, wobei das Öl typischerweise über eine poröse Walze 16 auf das Band 29 aufgetragen wird. Das Band 29 gleitet später dem Füllkeil 20 entlang, wo das über die Innenbeölung aufgetragene Öl als Gleitmittel wirkt und zur Abdichtung von Band 29 und Füllkeil 20 dient, damit keine Luftblasen ins Innere der Weichkapsel gelangen können.

Die Aussenseite des Bandes 29 wird in analoger Art über die Aussenbeölung ebenfalls mit einer porösen Walze 17 beölt. Diese Beölung verhindert, dass die frischen Kapseln 22 in den weiteren Verarbeitungsschritten aneinander kleben.

In Figur 2 wird zuerst die Innenseite des Bandes 29 beölt 16 und dann die Aussenseite 17. Dies ist jedoch nur eine Möglichkeit, auch die umgekehrte Reihenfolge ist möglich.

Ausserdem gibt es auf der Verfahrensstrecke von der Giesstrommel 13 bis zur zentralen Verkapselungseinheit eine Vielzahl von möglichen Anordnungen von verschiedenen Walzen und anderen Hilfsmitteln zur Führung des Bandes 29, wie zusätzliche Walzen, die aktiv angetrieben werden, Rollen, die nicht aktiv angetrieben werden oder Stege und Stäbe, über die das Band 29 geführt wird.

In der zentralen Rotary-Die-Verkapselungseinheit 44 werden die von beiden Seiten zugeführten Bänder 29 an den beiden Flanken eines Füllkeils 20 vorbei zwischen zwei Formwalzen 21 geführt, welche mit einer Mehrzahl von Formen 21a die Weichkapseln formen und ausstanzen. Durch taktweise durch den Füllkeil 20 eingespritztes flüssiges Füllgut 19 werden die Bänder 29 zu den zwei Seiten der jeweiligen entstehenden Weichkapseln geformt, wobei die Hülle aus zwei miteinander verschweissten Hälften besteht, die von den beiden Bändern stammen.

Die Geometrie der Weichkapseln wird durch die Formwalzen 21 mit ihren Formen 21a bestimmt, welche die einzelnen Kapseln schliesslich auch ausstanzen. Die Formwalzen haben einen Radius R2 und eine Oberflächengeschwindigkeit V2, welche über die Drehzahl der Formwalzen eingestellt wird. Die Produktionsgeschwindigkeit einer Anlage wird typischerweise mit der Drehzahl der Formwalzen charakterisiert.

Unterhalb Formwalzen 21 fällt ein Teil der frisch geformten Weichkapseln 22 hinunter, ein anderer Teil bleibt an den Formwalzen hängen und wird durch rotierende Bürsten abgebürstet (nicht dargestellt).

Ein dritter Teil der Weichkapseln bleibt in dem Netz (net) 23 hängen, das von den zwei aufeinanderliegenden Bändern 29, aus denen die Weichkapselhüllen ausgestanzt worden sind, übriggeblieben ist. Zwei Zugrollen (mangle rolls) 25 ziehen das Netz 23 des Doppelbandes mit den darin noch befindlichen Weichkapseln 22 nach unten und dehnen dasselbe beträchtlich, wodurch die meisten Weichkapseln 22 aus dem Netz 23 fallen.

Durch die zwei Abstreifrollen (stripper rolls) 24 werden schliesslich die letzten noch im Netz 23 hängen gebliebenen Weichkapseln 22 abgestreift.

Durch ein kurzes, erstes Förderband 26 werden die frischen Weichkapseln 22 aus der Verkapselungszone auf ein längeres, zweites Förderband 27 transportiert. Auf dem zweiten Förderband gelangen die frischen Weichkapseln 22 stromabwärts zum Förderkanal 5.

In der obigen Beschreibung der Verfahrensschritte und des Rotary-Die-Verfahrens sind die erfindungsgemässen Verbesserungen mit Ausnahme der Polierwalzen 14 nicht aufgeführt. Die Beschreibung liefert jedoch den Hintergrund für die Verbesserungen, die im Folgenden detailliert beschreiben werden.

### Modifizierung des Herstellungsverfahrens

Das erfindungsgemässe Verfahren beruht auf dem bekannten Rotary-Die-Verfahren, das in mehreren Punkten durch wesentliche und unerwartete Modifikationen erstaunlicherweise so deutlich verbessert werden konnte, dass das seit bald 100 Jahren bestehende und immer noch marktbeherrschende Verfahren zur Herstellung von Gelatine-Weichkapseln perfektioniert und wirtschaftlich deutlich übertroffen wird.

Insbesondere nutzt das erfindungsgemässe Verfahren das in WO 2010/100196 A1 beschriebenen Verfahren zur Herstellung von auf Stärke basierenden Weichkapseln, das als ein charakteristisches Merkmal eine Phasenumwandlung mittels Gelatinisierung der Stärke in der Giessmasse 11 auf einer geheizten Giesstrommel 13 (casting drum) einsetzt, um das für die Verkapselung benötigte Band 29 zu erhalten.

Die Verfahren aus dem Stand der Technik verwenden ausschliesslich die Phasenumwandlung mittels Gelierung eines Geliermittels auf einer gekühlten Giesstrommel, um aus einer flüssigen Giessmasse ein festes Band zu erhalten.

Der Begriff Gelatinisierung ist historisch über die Analogie zu Gelatine zustande gekommen und führt leider immer wieder zu Missverständnissen und zu hartnäckiger Verwirrung, weil man bei dem Begriff an Gelatine denkt. Allerdings hat «Gelatinisierung» stofflich nichts mit Gelatine zu tun.

Der Begriff Gelatinisierung wird ausschliesslich betreffend Stärke verwendet und betrifft die Phasenumwandlung von teilkristalliner Stärke, welche durch Erhitzen der Stärke in wässriger Umgebung ausgelöst wird. Dabei wird ein Zustand erhalten, der an ein Gel erinnert. Der Begriff Gel ist von Gelatine abgeleitet, da diese das bekannteste Geliermittel ist.

Es wurden erstaunlicherweise mehrere Verbesserungen gefunden, wobei jede insofern ganz unerwartet war, als der bekannte Stand der Technik keine Hinweise darauf liefert und diese Verbesserungen Aspekte des Verfahrens betreffen, die generell als nebensächlich gelten oder neu sind. Diese Verbesserungen betreffen:
1. Die Minimierung der Orientierung der Makromoleküle in dem Band 29, mit dem die Weichkapselhülle hergestellt wird, in dem Verfahrensbereich von Giesstrommel 13 bis Füllkeil 20, wobei diese Massnahme insbesondere durch eine Minimierung der Verstreckung des Bandes 29 in diesem Verfahrensbereich erreicht wird.
2. Die Beölung des Bandes 29, insbesondere der Seite des Bandes 29, welche bei der fertigen Kapsel die Aussenseite der Hülle bildet, und die vorteilhaft möglichst gering ist.
3. Die Temperaturführung des Bandes 29 vor der Verkapselung, des Bandes während der Verkapselung und der Kapsel nach der Verkapselung, wobei tiefe Temperaturen vorteilhaft sind.
4. Die Rezeptur der Giessmasse 11, aus der das Band 29 hergestellt wird, woraus sich für das Rotary-Die-Verfahren wichtige Eigenschaften ergeben, insbesondere die Festigkeit des Bandes 29, welche deutlich erhöht werden konnte, sowie die Verschweissbarkeit bei der Nahtbildung, welche verbessert werden konnte.

Mit der Anwendung von wenigstens einer dieser Verbesserungen kann das Verfahren bereits deutlich verbessert werden. Vorteilhaft werden mindestens zwei dieser Verbesserungen kombiniert, noch vorteilhafter drei, noch vorteilhafter alle vier.

Die Verbesserungen 1 und 2 sind generell bei Verfahren zur Herstellung von vegetarischen Weichkapseln vorteilhaft und führen insbesondere zu verbesserter Wirtschaftlichkeit des Herstellungsverfahrens.

Die Verbesserung 3 ist mindestens teilweise ebenfalls für vegetarische Weichkapseln vorteilhaft, insbesondere betreffend die Steuerung der Temperatur während und nach der Verkapselung.

Die Verbesserung 4 betrifft die Technologie, welche auf der Gelatinisierung der Stärke in der Giessmasse 11 auf einer geheizten Giesstrommel 13 basiert.

Die Verbesserungen wirken einerseits additiv, andererseits auch synergistisch. Durch eine höhere Festigkeit des Bandes 29 kann die Verstreckung beispielsweise in grösserem Umfang reduziert werden. Durch eine minimale Aussenbeölung muss die Verstreckung weniger stark minimiert werden, in der Kombination werden jedoch besonders gute Verschweissungen der beiden Kapselhüllenhälften (Nähte) erhalten. Durch eine tiefe Temperatur des Bandes 29 bei der Verkapselung muss die Aussenbeölung weniger minimiert werden, in der Kombination jedoch werden dann besonders gute Nähte erhalten.

Je höher die Produktionsgeschwindigkeit, in umso grösserem Umfang mehr sind die einzelnen Verbesserungen umzusetzen und umso vorteilhafter ist eine Kombination von zwei, drei oder sogar aller dieser Verbesserungen.

Die verschiedenen Massnahmen zur Verbesserung der Weichkapselproduktion ermöglichen auch eine Flexibilität, denn das erfindungsgemässe Verfahren soll auf verschiedenen in der Weichkapselbranche bestehenden Rotary-Die-Vorrichtungen 4 und in den verschieden konfigurierten Umgebungen betreffend die weiteren Verfahrensschritte umsetzbar sein.

Für die Qualifizierung der Produktionsgeschwindigkeit beim Rotary-Die-Verfahren wird typischerweise die Drehgeschwindigkeit der Formwalze 21 herangezogen. Während die Produktionsgeschwindigkeit bei Gelatine-Weichkapseln im Bereich um 4 U/min liegt und bei alternativen vegetarischen Weichkapseln typischerweise nur um 2 bis 3 U/min gefahren werden, können aufgrund der Verbesserungen der vorliegenden Erfindung, insbesondere betreffend das auf der Gelatinisierung von Stärke basierende Verfahren sogar Produktionsgeschwindigkeiten von mehr als 4 U/min, insbesondere bis 5 U/min, teilweise sogar mehr erreicht werden und dies mit einem flexiblen, robusten Prozess, der konstant qualitativ hochwertige Kapseln liefert.

In dem dargestellten erfindungsgemässen Verfahren wird die Giesstrommel 13 geheizt, und nicht wie bisher bekannt gekühlt.

Auf der geheizten Giesstrommel 13 wird die zunächst noch flüssige Giessmasse 11 durch eine Phasenumwandlung in ein festes, elastisches Band 29 umgewandelt.

Die Temperaturerhöhung der Giessmasse 11 auf den auf rund 100 °C geheizten Giesstrommeln 13 führt zu einer Gelatinisierung der in der Giessmasse 11 vorliegenden vorwiegend noch doppelbrechenden, teilkristallinen Stärkekörner der granulären Stärke.

Die Gelatinisierung ist eine für Stärke charakteristische Phasenumwandlung, wobei die kristalline Struktur der Stärkekörner verschwindet und diese Körner unter Aufnahme des Wassers und des Weichmacher massiv quellen, was zum Verschwinden der flüssigen Phase und zur Umwandlung der viskosen Giessmasse 11 in einen mehr oder weniger festen, elastischen Zustand führt. Es entsteht das für die Verkapselung benötigte Bandes 29.

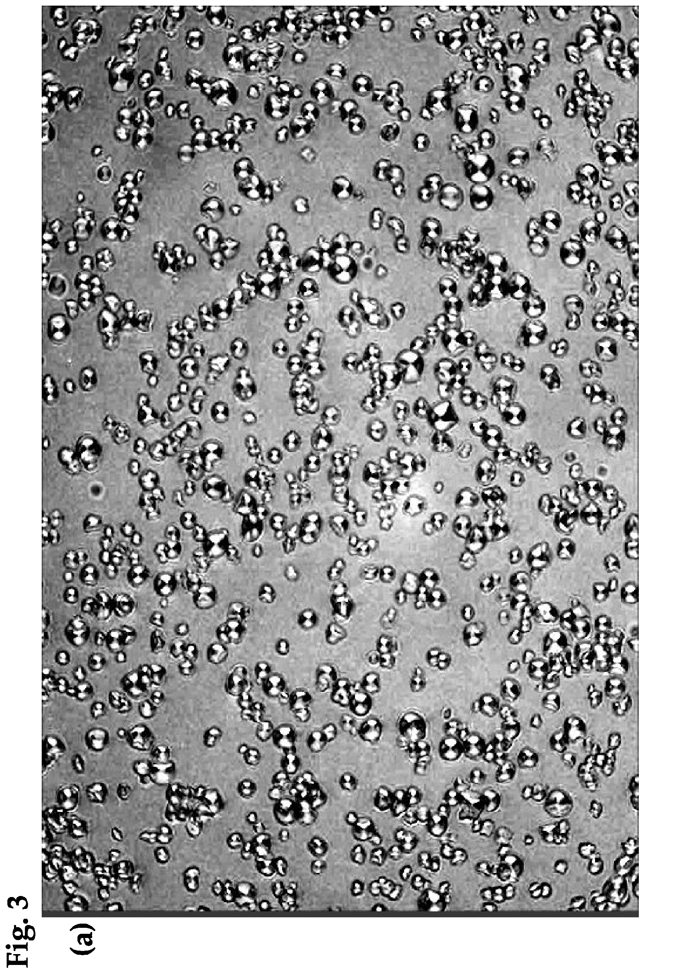

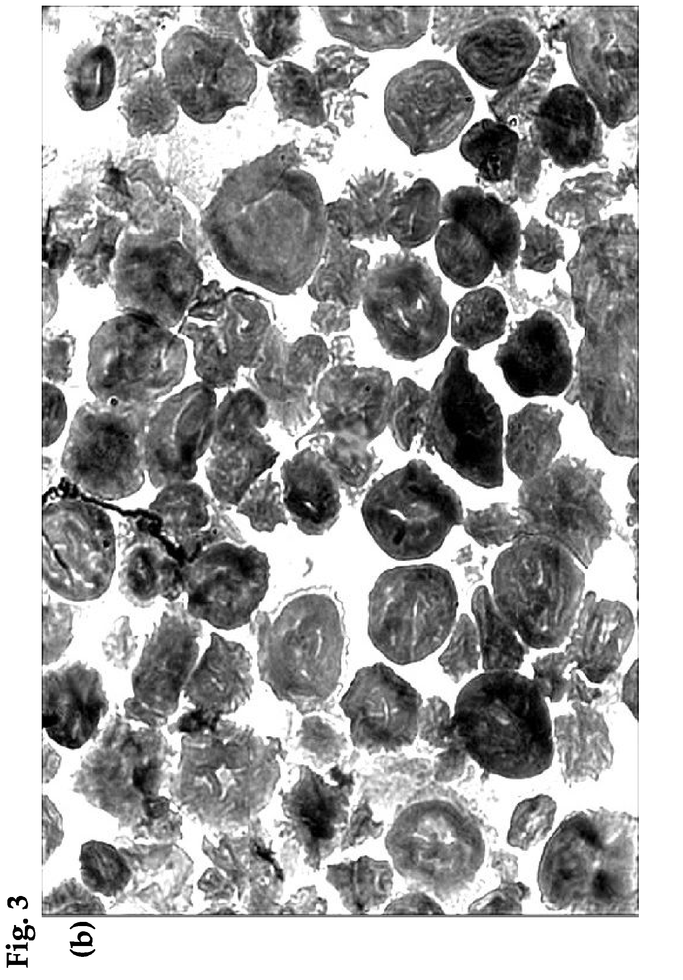

Die Mikrostruktur dieses Bandes 29 kann im Lichtmikroskop untersucht werden, wie dies mit Figur 3 gezeigt wird.

Figur 3(a) zeigt eine lichtmikroskopische Aufnahme, einer wässrigen Suspension von unverarbeiteter, nativer, granulärer Tapioka-Stärke. Die vor allem bei den grösseren Stärkekörnern sichtbare, sogenannte Malteserkreuz-Struktur ist ein typisches Identifikationsmerkmal von nativer, granulärer Stärke und ist eine Folge der Kristallinität solcher Stärke-Körner. In diesem Zustand verhalten sich die Stärkekörner als feste, harte Partikel, die in kaum Wasser aufnehmen und so in der Giessmasse 11 suspendiert werden können.

Figur 3(b) zeigt eine lichtmikroskopische Aufnahme derselben Tapioka Stärke, wobei die in der Giessmasse 11 suspendierten Stärkekörner durch die Temperaturerhöhung auf der Giesstrommel 13 gelatinisiert worden sind. Dabei haben die Stärkekörner ihre teilkristalline Struktur vollständig verloren, und die flüssige Phase von Wasser und Weichmacher aufgenommen, wodurch sie massiv an Grösse zugenommen haben. Dadurch ist die zuvor flüssige Giessmasse 11 zu einem mehr oder weniger festen Band 29 geworden, das aus einem Agglomerat von miteinander verbundenen gelatinisierten Stärkekörnern besteht.

Für diese Aufnahme wurde ein Stück einer Weichkapselhülle in Wasser gequollen, sodass es deutlich weicher ist. Anschliessend wurde dieses aufgequollene Stück der Weichkapselhülle zwischen zwei Objektträgern gequetscht, damit es für die Mikroskopie untersuchbar wurde.

Die im Bild sichtbaren rundlichen Strukturen sind gelatinisierte Stärkekörner, die im Vergleich zu den nativen Stärkekörnern in Figur 3(a) durch die Gelatinisierung viel grösser geworden sind, und die Kristallinität und damit auch die Malteserkreuz-Struktur vollständig verloren haben. Durch die Probenpräparation sind die gelatinisierten Stärkekörner teilweise etwas voneinander separiert und damit besser sichtbar gemacht worden.

Im Band und in den Hüllen der Stärke-Weichkapseln sind diese gelatinisierten Stärkekörner dicht aneinanderliegend und miteinander verbunden.

Die im Bild dargestellten gelatinisierten Stärkekörner können durch definierte Wiedergewinnungsverfahren auch quantitativ aus dem Band und aus der Weichkapselhülle zurückgewonnen werden. Die Breite des Bildes entspricht hier ebenfalls 570 µm und die gelatinisierten Stärkekörner haben eine Grösse im Bereich von 50 µm.

### Orientierung der Makromoleküle im Band

Es ist im Stand der Technik üblich, dass das Band 29 auf der Stecke von der Giesstrommel 13 bis zur Rotary-Die-Verkapselungseinheit verstreckt wird. Damit wird die Führung des Bandes 29 auf dieser Stecke erleichtert, das Band 29 ist straff gespannt und hängt zwischen den Walzen nicht durch. Am Füllkeil 20 wird ein guter Einzug des Bandes 29 erhalten. Bei der Gelatine wird das Band 29 grosszügig verstreckt und das Ausmass dieser Verstreckung, die deutlich oberhalb von 20% liegt, hat soweit keinen Einfluss auf die Nahtbildung zwischen den Kapselhälften.

Die Naht ist die rund um die Weichkapsel laufende Zone, wo die beiden Hälften der Kapselhülle miteinander verbunden sind. Eine gute, dicke Naht ist entscheidend für eine gute Kapselqualität. Die Kapselqualität wird in erster Linie durch die Leck-Rate (leaker rate), den Anteil undichter Kapseln, bestimmt, der natürlich möglichst gering sein soll.

Die totale Verstreckung S des Bandes 29 von der Giesstrommel 13 bis zum Füllkeil 20 wird durch die Differenz der Oberflächengeschwindigkeit V1 der Giesstrommel 13 und der Oberflächengeschwindigkeit V2 der Formwalzen bestimmt:$Verstreckung in % : S = 100 * \left(V2-V1\right) / V 1$

V1 in cm/min ergibt sich aus der Drehzahl der Giesstrommel 13 (Umdrehungen pro Minute, U/min) multipliziert mit dem Umfang der Giesstrommel 13 (in cm), und V2 in cm/min ergibt sich analog aus der Drehzahl der Formwalzen multipliziert mit dem Umfang der Formwalzen (in cm).

Bei den vegetarischen Alternativen zur Gelatine wurden im Stand der Technik die üblichen hohen Verstreckungsgrade übernommen, weil sich das bei der Gelatine bewährt hatte und damit eine gute Filmführung erreicht werden kann. Ausserdem sind die Bänder bei vegetarischen Alternativen vergleichsweise sehr weich, so dass die Bandführung durch eine hohe Verstreckung erleichtert wird.

Erstaunlicherweise wurde nun aber gefunden, dass dieser Verstreckungsgrad S ein empfindlicher Parameter mit grossem Einfluss auf die Robustheit des Herstellungsprozesses und die Kapselqualität ist.

Dies war sehr überraschend, da das Band 29 in der zentralen Verkapselungseinheit bei der Kapselbildung um rund 60% gedehnt, also um mehr als die Hälfte verlängert wird. Es war nicht zu erwarten, dass eine vorausgehende, viel geringere Verstreckung einen Einfluss auf die Qualität der Nahtbildung haben sollte.

Ausserdem ist das Band 29 nicht voll elastisch, sondern auch plastisch. Das Material fliesst etwas, d.h. eine Dehnung entspannt sich wieder von selbst, das Band 29 «vergisst», dass es vorher gedehnt worden ist. Und trotzdem hat eine solche Vordehnung einen grossen Einfluss auf die Nahtqualität.

Die Erklärung ist auf molekularer Ebene zu suchen, wo die Makromoleküle im Band infolge der Vordehnung des Bandes zwischen Giesstrommel und Formwalzen bis zu einem gewissen Grad orientiert werden. Diese Orientierung wird durch die Plastizität bis zur Verkapselung nicht völlig abgebaut und führt dort dazu, dass das Band 29 etwas fester ist und somit eine etwas grössere Spannung auf die frische Naht zwischen den Kapselhälften einwirkt und diese schwächt. Diese Schwächung der Naht kann minimiert und praktisch ausgeschaltet werden, indem eine Orientierung der Makromoleküle im Band 29 auf dem Weg von der Giesstrommel bis zur zentralen Verkapselungseinheit möglichst verhindert, oder zumindest minimal gehalten wird.

Dies kann vorteilhaft mit dem Verstreckungsgrad S erreicht werden, mit dessen Minimierung die verbleibende Orientierung direkt vor der Verkapselung minimal wird.

Ausserdem wird eine Orientierung der Makromoleküle vorteilhaft minimal gehalten oder verhindert, indem das Band 29 möglichst wenig auf der Giess-Trommel klebt.

Weiter kann vorteilhaft auch die Temperaturführung des Bandes 29 so gesteuert werden, dass eine gewisse Orientierung der Makromoleküle durch die Wärmebewegung der Makromoleküle wieder aufgelöst wird. Vorteilhafte Temperaturen werden weiter unten bei den Ausführungen zur Temperatur des Bandes 29 spezifiziert.

Die Verstreckung S des Bandes 29 im Verfahrensabschnitt ab Giesstrommel 13 bis Formwalze 21 ist daher vorteilhaft nicht grösser als 20%, noch vorteilhafter nicht grösser als 17%, noch vorteilhafter nicht grösser als 15%, noch vorteilhafter nicht grösser als 13%, noch vorteilhafter nicht grösser als 11%, noch vorteilhafter nicht grösser als 9%, noch vorteilhafter nicht grösser als 8%, noch vorteilhafter nicht grösser als 7%, noch vorteilhafter nicht grösser als 6%, noch vorteilhafter nicht grösser als 5%, und noch vorteilhafter nicht grösser als 4%

Die Verstreckung S wird durch die Drehzahlen der Giesstrommel 13 und der Formwalzen eingestellt.

Bei sehr tiefen Verstreckungen und hohen Produktionsgeschwindigkeiten kann das Kleben des Bandes 29 auf der Giesstrommel 13 dem Ziel einer minimalen Verstreckung hinderlich werden, weil zum Abziehen des Bandes 29 grössere Kräfte benötigt werden, die dann eine höhere Verstreckung bedingen.

In einer vorteilhaften Ausführung des Herstellungsverfahrens wird daher das Kleben des Bandes 29 auf der Giesstrommel 13 minimiert.

In einer vorteilhaften Ausführung wird die Giesstrommel 13 wiederholt gereinigt, noch vorteilhafter kontinuierlich gereinigt, und noch vorteilhafter, indem sie kontinuierlich mit einer Polierwalze 14 gereinigt wird. Dazu kann ein textiles Material wie ein Poliertuch verwendet werden, insbesondere ein Poliertuch, das keine Fasern verliert. Vorteilhaft wird die Polierwalze 14 aktiv angetrieben, vorteilhaft mit einer einstellbaren Drehzahl. Die Polierwalze 14 kann sich gleichsinnig mit der Giesstrommel 13 oder gegensinnig drehen.

In einer anderen vorteilhaften Ausführung wird die Giesstrommel 13 mit einer Beschichtung modifiziert, welche die Haftung reduziert. Hierzu kommen die gemäss dem Stand der Technik bekannten, die Haftung reduzierende Materialien in Frage wie beispielsweise verschiedene PTFE- Kunststofftypen.

Die Verstreckung insgesamt wird wie beschrieben, in Abhängigkeit der Geometrie von Giesstrommel und Formwalzen, durch die Drehzahlen von Giesstrommel 13 und Formwalzen 21 bestimmt und über diese Parameter eingestellt.

Die beiden Giesstrommeln 13 werden typischerweise je von einem eigenen Motor angetrieben. Die beiden Formwalzen werden typischerweise von einem einzigen Motor angetrieben.

Die zwischen Giesstrommel 13 und Formwalzen liegenden intermediären Walzen werden typischerweise von einem separaten Motor angetrieben. Die Drehzahl dieses Motors ist vorteilhaft so einzustellen, dass die resultierenden Verstreckungen, die bei den einzelnen intermediären Walzen erfolgen, kleiner sind als die Verstreckung insgesamt.

In einer vorteilhaften Ausführung gibt es bei den intermediären Walzen mindestens zwei Gruppen, die unterschiedliche Oberflächengeschwindigkeiten aufweisen, sodass eine erste Verstreckung von der Giesstrommel 13 zu der ersten Gruppe vorliegt und eine zweite Verstreckung von der ersten Gruppe zu der zweiten Gruppe, gefolgt von einer Verstreckung von der zweiten Gruppe zu der Formwalze. Somit nimmt die Verstreckung auf dem Weg von der Giesstrommel 13 bis zu den Formwalzen vorteilhaft in mehreren Etappen zu.

Ein tiefer Verstreckungsgrad führt zu besonders guten und stabilen Nähten, jedoch wird dabei die Bandführung erschwert, insbesondere bei hohen Produktionsgeschwindigkeiten.

In einer vorteilhaften Ausführung wird daher pro Band 29 mindestens eine Dämpfungswalze oder mindestens ein Dämpfungssteg, dieser ausgeführt beispielsweise als Stab, eingesetzt, welche die Bandführung stabilisiert.

In einer vorteilhaften Ausführung werden mit der Dämpfungswalze bzw. mit dem Dämpfungssteg Vibrationen auf das Band 29 übertragen, welche das Band 29 stabilisieren.

In einer vorteilhaften Ausführung wird pro Band 29 mindestens eine Vorrichtung eingesetzt, welche Vibrationen in transversaler Richtung auf das Band 29 überträgt, d.h. senkrecht zur Bandlaufrichtung und in der Ebene des Bandes 29. Dies kann beispielsweise ausgeführt werden durch eine stabförmige Vorrichtung in transversaler Richtung mit einer Achse parallel zu den Walzen

Der Einzug des Bandes 29 unter den Füllkeil 20 ist kritisch für das Herstellungsverfahren. Wenn das Band 29 an dieser Stelle nicht hinreichend gerade ist, kann es zum Einzug von Luft kommen, welche dann als Luftblasen in den Weichkapseln vorliegt. Solcherart fehlerhafte Weichkapseln müssen dann aussortiert werden.

In einer vorteilhaften Ausführung wird daher in der Verfahrensstrecke der letzten 15cm vor dem Einzug des Bandes 29 unter den Füllkeil 20 eine Dämpfungswalze und/oder ein Dämpfungssteg eingesetzt, welche vorteilhaft mit Vibrationen angeregt werden, und/oder eine Vorrichtung für Vibrationen in transversaler Richtung.

### Aussenbeölung

So wie ein grosszügiger Verstreckungsgrad bei Gelatine-Weichkapseln selbstverständlich ist und für die vegetarischen Alternativen übernommen wurde, wird im Stand der Technik bei Gelatine-Weichkapseln das Band 29 auf beiden Seiten grosszügig beölt und wurde auch diese Verfahrensmassnahme für die vegetarischen Alternativen übernommen. Die auf dem Markt erhältlichen Rotary-Die-Anlagen sind mit Pumpen ausgerüstet, welche das Öl zur Beölung des Films 28 zu den porösen Walzen pumpen und durch die Poren hindurch, sodass das Öl kontinuierlich auf das Band 29 aufgetragen wird, bis es davon heruntertropft. Die Aussenbeölung ölt die Seite des Bandes 29, die schliesslich die Aussenseite der Kapselhüllen bildet. Die Innenbeölung ölt die Seite des Bandes 29, die schliesslich die Innenseite der Kapselhülle bildet. Bei der Innen- und Aussenbeölung wird das Öl typischerweise in einer Menge im Bereich von 1.5 g/m² dosiert. Dies entspricht einem Ölfilm der Dicke von nur etwa 1.5 µm oder weniger.

Nun wurde überraschenderweise gefunden, dass das Ausmass der Aussenbeölung ein wichtiger und empfindlicher Parameter ist im Hinblick auf Robustheit und Qualität ist, insbesondere bei hoher Produktionsgeschwindigkeit. Es ist in der Tat erstaunlich, dass es eine sehr wichtige Rolle für die Qualität der Nähte spielt, ob der Ölfilm der Aussenbeölung 1.5 µm dick ist oder 1.0 oder 0.5 µm oder noch weniger.

Es wurde weiter gefunden, dass ohne Aussenbeölung die besten Kapselnähte erhalten werden.

Da die Aussenbeölung jedoch nach der Verkapselung notwendig ist, damit die frischen Kapseln 22 nicht an der Formwalze 21 und aneinander kleben, wurden zwei Verfahrensvarianten entwickelt, um den wichtigen Einfluss der Aussenbeölung auf die Qualität der Nahtbildung und damit auf die Leck-Rate optimal zu nutzen. Mit anderen Worten wird die Menge und/oder der Bereich der Beölung abhängig von der Produktionsgeschwindigkeit und der konkret zu fertigenden Produkte gesteuert und/oder geregelt. Dies wird über eine Kontrolleinheit K geregelt (Fig. 2).

In einer vorteilhaften Ausführung wird das Band 29 aussen beölt, jedoch liegt die dosierte Ölmenge bei höchstens 2,0 g/m², vorteilhaft bei höchstens 1.5 g/m², vorteilhaft bei höchstens 1,0 g/m², vorteilhaft bei höchstens 0,7 g/m², noch vorteilhafter bei höchstens 0,55 g/m², noch vorteilhafter bei höchstens 0,45 g/m², noch vorteilhafter bei höchstens 0,35 g/m², noch vorteilhafter bei höchstens 0,25 g/m², noch vorteilhafter bei höchstens 0,20 g/m², und noch vorteilhafter bei höchstens 0,15 g/m².

In einer weiteren vorteilhaften Ausführung findet noch eine zusätzliche Aussenbeölung statt, jedoch erst nachdem die Kapseln gebildet worden sind.

Vorteilhaft findet diese zusätzliche Aussenbeölung auf der Verfahrensstecke nach dem langen Förderband bis zur Vorkonditionierung 6 statt, wo die Kapseln über einen Luftstrom durch einen Förderkanal 5, beispielsweise durch ein Rohr, zu der Vorkonditionierung 6 geführt werden. Hier kann die zusätzliche Aussenbeölung beispielsweise so erfolgen, dass das zusätzliche Öl in diesen Luftstrom dosiert wird, wo es infolge des Luftstroms zu einer guten Verteilung auf den Kapseloberflächen kommt.

In einer weiteren vorteilhaften Ausführungsvariante findet bis zur Bildung der Kapseln keine Aussenbeölung statt. Vorteilhaft wird dann nach der Kapselbildung eine Aussenbeölung durchgeführt, insbesondere wie im vorangehenden Absatz beschreiben.

In einer vorteilhaften Ausführung wird die Aussenbeölung so dosiert, dass die Kapseln später nicht entölt werden müssen.

### Temperaturführung

Auch die Temperatur des Bandes 29, mit dem verkapselt wird, sowie die Temperatur von Füllkeil 20 und Formwalzen 21 haben überraschenderweise einen Einfluss auf Robustheit und Qualität des Verfahrens, insbesondere bei hohen Produktionsgeschwindigkeiten.

Die frischen, noch sehr weichen Weichkapseln 22 sind zwar sehr robust, man kann sie verdrehen und damit massiv mechanisch beanspruchen, ohne dass sie Schaden nehmen. Andererseits sind sie erstaunlicherweise auch ohne mechanische Belastung sehr empfindlich bei der Schweissnaht, wenn sie längere Zeit eine erhöhte Temperatur aufweisen. Das heisst, es können Lecks in der Schweissnaht entstehen, weshalb vorteilhaft höhere Temperaturen zu vermeiden sind und eine Kühlung vorteilhaft ist.

Insgesamt betreffen die Massnahmen zur Temperaturführung vor allem verschiedene Kühlmöglichkeiten, die umso wichtiger sind, je höher die Produktionsgeschwindigkeit ist. Insbesondere wird im Unterschied zum Stand der Technik neben einer passiven Kühlung bei vorteilhaften Ausführungsformen auch eine aktive Kühlung durch gekühlte Kontaktflächen, Luftdüsen, Kühlleitungen oder entsprechende technische Mittel eingesetzt.

Vorteilhafterweise werden fördertechnisch das Band begleitende Kühlelemente 38.1 (insbesondere aktiv gekühlte Rollen oder Gurtbänder) entlang dessen Förderstrecke eingesetzt.

Diese begleitenden Kühlelemente 38.1 werden zur Erreichung einer stabilen Prozessumgebung durch die Kontrolleinheit K (Fig. 2) vorteilhaft geregelt oder gesteuert, um für das Band 29 die gewünschte Kühltemperatur entlang des Förderwegs zu bewirken.

Einerseits kann die Empfindlichkeit der Schweissnaht bei erhöhter Temperatur reduziert werden, wenn die Verkapselung bereits bei nicht zu hohen Temperauren erfolgt. Andererseits wird eine gewisse minimale Temperatur für eine gute Nahtbildung benötigt und ebenfalls im Hinblick auf die möglichst geringe Orientierung der Makromoleküle im Band 29 sollte die Temperatur des Bandes 29 nicht zu tief sein, weil die Orientierung der Makromoleküle bei höherer Temperatur schneller abgebaut werden.

Daher ist eine ausgewogene Kühlung bis zur Bildung der Kapsel, wie auch eine Kühlung nach Bildung der Kapsel vorteilhaft.

Die hinsichtlich der Temperatur bei der Kapselbildung und der Temperatur der frischen Kapsel 22 optimalen Bedingungen werden durch eine Kombination von verschiedenen Massnahmen erhalten, womit eine optimale Steuerung des Temperaturverlaufs des Verfahrens und damit eine optimale Robustheit der frischen Kapseln 22 erreicht wird.

Die Steuerung des Temperaturverlaufs des Verfahrens wird durch die Steuerung der Temperatur des Bandes 29 vor dem Füllkeil 20, der Temperatur des Füllkeils 20, der Temperatur der Formwalzen, der Temperatur des Füllguts 19, und der Temperatur der Kapselhülle unmittelbar nach der Verkapselung erreicht, sowie mit auf die Verkapselung nachfolgenden Massnahmen.

Die verschiedenen Massnahmen wirken additiv wie auch synergistisch in dem Sinne dass beispielsweise eine ausgeprägte Kühlung des Bandes 29 vor dem Füllkeil 20 eine weniger ausgeprägte Kühlung bei den nachfolgenden Arbeitsschritten ermöglich, andererseits ist die Kombination besonders vorteilhaft, vor allem bei hohen Produktionsgeschwindigkeiten. Wenn durch entsprechende Massnahmen die Temperatur der Weichkapseln direkt nach der Verkapselung bereits im Bereich der Raumtemperatur erhalten wird, sind nachfolgende Massnahmen zur Senkung der Temperatur nicht mehr notwendig. Andererseits sind diese dann besonders wichtig, wenn die Massnahmen zur Kühlung des Bandes 29 vor und bei der Verkapslung nicht oder nur in geringem Umfang umgesetzt wurden. Damit kann flexibel auf verschiedene Bedingungen, wie sie bei den verschiedenen industriellen Weichkapsel-Herstellungslinien bestehen, eingegangen werden.

### Temperatur des Bandes

Die Giesstrommel 13 hat eine Oberflächentemperatur von rund 100 °C und mit etwa dieser Temperatur verlässt das Band 29 die Giesstrommel 13. Die Temperatur des Bandes 29 sollte dann vor dem Füllkeil 20 vorteilhaft nicht höher sein als 60 °C.

Die Zeit, die das Band 29 von der Giesstrommel 13 bis zum Füllkeil 20 unterwegs ist, hängt stark ab von der Drehzahl der Formwalzen und deren Durchmesser, sowie von der Länge des Bandes 29 auf der Verfahrensstrecke von der Giesstrommel 13 bis zum Füllkeil 20. Diese Zeit liegt typischerweise im Bereich von etwa 10 bis 70 Sekunden. Bei tiefen Drehzahlen und somit längeren Zeiten ist die passive Kühlung des Bandes 29 durch die Umgebungsluft auf eine Temperatur von etwa 60 °C gut möglich.

Bei höheren Produktionsgeschwindigkeiten und Drehzahlen der Formwalzen von etwa 4 U/min und mehr, wäre das Band 29 bei der Verkapselung zu heiss. Und vorteilhaft sollte die Temperatur des Bandes 29 vor dem Füllkeil 20 tiefer sein als 60 °C. Darum ist eine Kühlung des Bandes 29 vorteilhaft.

In einer vorteilhaften Ausführung liegt die Temperatur des Bandes 29 vor dem Füllkeil 20, gemessen mit einem IR-Thermometer bei höchstens 60 °C, vorteilhaft bei höchstens 55 °C, noch vorteilhafter bei höchstens 50 °C, noch vorteilhafter bei höchstens 45 °C, noch vorteilhafter bei höchstens 40 °C, noch vorteilhafter bei höchstens 35 °C, und noch vorteilhafter bei höchstens 31 °C.

Bei zu tiefer Temperatur des Bandes 29 werden bestehende Orientierungen der Makromoleküle im Band 29 weniger abgebaut und wird die Verschweissung der Naht weniger gut, weshalb das Band 29 vor dem Füllkeil 20 nicht zu kalt sein sollte. In einer vorteilhaften Ausführung liegt daher die Temperatur des Bandes 29 vor dem Füllkeil 20, gemessen mit einem IR-Thermometer bei nicht weniger als 15 °C, noch vorteilhafter bei nicht weniger als 19 °C. noch vorteilhafter bei nicht weniger als 21 °C, noch vorteilhafter bei nicht weniger als 23 °C, noch vorteilhafter bei nicht weniger als 25 °C, und noch vorteilhafter bei nicht weniger als 27 °C.

Das Band 29 hat unmittelbar nach der Giesstrommel 13 eine Temperatur im Bereich von etwa 100 °C. Die Temperatur des Bandes 29 vor dem Füllkeil 20 wird in einer vorteilhaften Ausführung durch eine Kühlung erhalten, wobei die folgenden Kühlmethoden einzeln oder in Kombination eingesetzt werden können.

In einer vorteilhaften Ausführung ist der Weg des Bandes 29 von der Giesstrommel 13 bis zum Füllkeil 20 bei der eingesetzten Rotary-Die-Anlage genügend lang für eine passive Kühlung des Bandes 29 auf die gewünschte Temperatur vor dem Füllkeil 20.

Nötigenfalls wird in einer vorteilhaften Ausführung dieser Weg so weit verlängert wird, dass die gewünschte Kühlung durch die Kühlung der Atmosphäre erhalten werden kann. Die Verlängerung des Weges kann beispielsweise durch Umlenkrollen erhalten werden.

Insbesondere bei hohen Produktionsgeschwindigkeiten ist jedoch die erforderliche Kühlstrecke zu gross, als dass eine passive Kühlung durch die Umgebungsluft auf Raumtemperatur ausreichend sein kann. Darum wird in einer vorteilhaften Ausführung die Kühlung durch Luft, die auf das Band 29 geblasen wird, erhalten. Vorteilhaft hat diese Luft eine Temperatur von weniger als 25 °C, noch vorteilhafter weniger als 23 °C, noch vorteilhafter weniger als 21 °C, noch vorteilhafter weniger als 19 °C, und noch vorteilhafter weniger als 17 °C.

Diese Luftstromkühlung kann einseitig des Bandes 29, beidseitig, an einer oder an mehreren Stellen oder über einen oder mehrere längere Bereiche von der Giesstrommel 13 bis zum Füllkeil 20 erfolgen.

In einer weiteren vorteilhaften Ausführung erfolgt die Kühlung des Bands 29 durch mindestens eine gekühlte Walze im Bereich der intermediären Walzen zwischen Giesstrommel 13 und Füllkeil 20.

Vorteilhafter werden mindestens zwei Walzen gekühlt. Die betroffenen Walzen werden dabei vorteilhaft auf eine Temperatur von weniger als 25 °C, noch vorteilhafter weniger als 23 °C, noch vorteilhafter weniger als 21 °C, noch vorteilhafter weniger als 20 °C, noch vorteilhafter weniger als 19 °C, und noch vorteilhafter weniger als 17 °C gekühlt.

In einer vorteilhaften Ausführung werden die Kühlwalzen mit Wasser gekühlt.

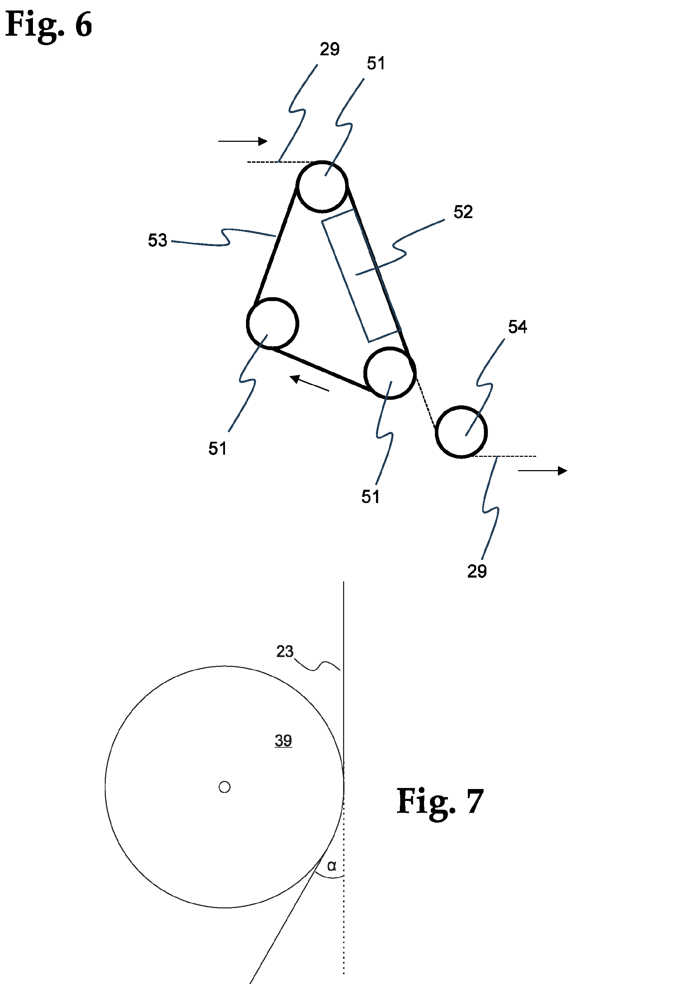

Eine weitere vorteilhafte Möglichkeit, das Band auf dem Weg zur Rotary-Die-Verkapselungseinheit zu kühlen, ist in Figur 6 gezeigt.

Über drei Rollen 51 ist ein Kühlband 53 geführt bzw. gespannt, vorteilhaft aus einem Material mit hoher Wärmeleitfähigkeit, beispielsweise aus Metall. Das Kühlband ist über einen Antrieb (nicht dargestellt) der Rollen 51 angetrieben.

Das Band 29 wird von rechts her zugefördert, und unter Kontakt oder in minimalem Abstand über das Kühlband 54 geführt, und anschliessend durch eine Führungsrolle 54 weitergeführt.

Das Kühlband 53 wird über eine Kühleinheit 52 kontinuierlich gekühlt, wobei das Kühlband in direktem Kontakt über die Kühleinheit 52 gleitet. Das Band 29 steht in direktem Kontakt zum Kühlband 53 und indirekt zur darunter gelegenen Kühleinheit 52. Die Kontaktfläche, und damit die Kontaktzeit, ist wegen des ebenen Kühlbandes höher als bei einer Kühlrolle. Dabei muss die Kühleinheit nicht zwingend direkt unter dem Band 29 angeordnet sein, sondern kann bspw. im Bereich oder der linken Rolle 51 angeordnet oder direkt in einer der Rollen 51 integriert sein.

Da sich das Kühlband 53 in gleicher Richtung bewegt wie das Band 29, ist die Gleitreibung zwischen Ihnen geringer als bei einer statischen Fläche.

Vorteilhaft sind die Fördergeschwindigkeiten des Kühlbands 53 und des Bands 29 leicht verschieden, so dass ein unerwünschtes Ankleben vermieden oder reduziert wird.

Diese beschriebene Kühlvorrichtung erlaubt eine rasche Kühlung des Bandes 29 auf die gewünschte Temperatur.

Die Kühlvorrichtung kann so als das Band 29 begleitende Kühlung eingesetzt werden und es können mehrere solche Kühlungen entlang der Förderung des Bandes 29 angeordnet werden. Im letzteren Fall kann es vorteilhaft sein, eine (zentrale) Kühleinheit 52 vorzusehen, welche über mit Kühlflüssigkeit gefüllte Rohrverbindungen die mehreren begleitenden Kühlungen stetig gekühlt hält. Die Kontrolleinheit K steuert oder regelt vorteilhafterweise diese eine oder mehreren Kühleinheiten 52 auf die gewünschte Prozesstemperatur für das Band 29.

Hierbei können Kühlbänder 53 zusätzlich auch eine Stützfunktion für das Band 29 ausüben in Bereichen, wo dieses stark durchhängt oder unerwünscht ausgebaucht ist, um auf diese Weise unerwünschte bzw. übermässige Verstreckungen in entsprechenden Förderabschnitten zu reduzieren oder zu vermeiden. Je nach konkreter Prozessumgebung kann dabei auch auf eine Kühlung verzichtet werden, so dass die Bänder 53 dann als reine Stützbänder (ohne Kühlung) wirken.

Bei besonderen Ausführungsformen entfallen die Kühleinheiten 52 somit ganz und die Kühlbänder 53 sind dann reine fördertechnische das Band 29 begleitende Stützbänder, die mit gleicher oder leicht versetzte Geschwindigkeit mit diesen mitlaufen.

Auf der Verfahrensstrecke von Giesstrommel bis Füllkeil gibt es je nach Anlagetyp auch eine oder mehrere Walzen oder Rollen, die nicht aktiv angetrieben werden. In den Figu-ren 5 und 5a ist beispielsweise die Führungswalze 35 vor dem Füllkeil nicht aktiv angetrieben und in den Figuren 4 und 4a sind die Führungswalzen 33 und 34 nicht aktiv angetrieben. Die genannten Walzen können vorteilhaft über ein Stützband mit einer aktiv angetriebenen Walze verbunden werden, wodurch ein Durchhängen vermieden werden kann. Zusätzlich muss die Kraft, um die nicht angetriebenen Walzen anzutreiben, nicht vom Band 29 übernommen werden, womit eine Orientierung der Makromoleküle im Band reduziert werden kann.

Um ein Verkleben der Kühl- oder Stützbänder 53 mit dem Band 29 bzw. dessen Adhäsion zu reduzieren, können diese aus einem antihaftwirkenden Material (z.B. Silikon) gefertigt sein oder auch abperlend (hydrophob/oleophob) oberflächenbeschichtet sein.

Kumulativ oder alternativ können diese einen netzartigen Aufbau haben oder eine Textur besitzen, die antihaftend ist und vorteilhafterweise insbesondere eine Noppentopologie besitzt («Lotoseffekt»).

Alternativ oder ergänzend sind die Kühl- oder Stützbänder 53 gleitlackbeschichtet oder sind antistatisch.

### Temperatur des Füllkeils

Eine weitere Temperierung des Bandes 29 unmittelbar vor und während der Verkapselung kann mit der Temperatur des Füllkeils 20 erfolgen, wobei die Temperatur des Füllkeils 20 mit einem Thermoelement 37 gemessen werden kann, welches sich im Inneren des Füllkeils 20 befindet.

In einer vorteilhaften Ausführung liegt die Temperatur des Füllkeils 20 bei höchstens 60 °C, noch vorteilhafter bei höchstens 55 °C, noch vorteilhafter bei höchstens 50 °C, noch vorteilhafter bei höchstens 45 °C, noch vorteilhafter bei höchstens 40 °C, noch vorteilhafter bei höchstens 35 °C, und noch vorteilhafter bei höchstens 31 °C.

Bei zu tiefer Temperatur des Füllkeils 20 wird die Nahtbildung beeinträchtigt, weshalb in einer vorteilhaften Ausführung die Temperatur des Füllkeils 20 nicht tiefer ist als 10 °C, noch vorteilhafter nicht tiefer als 14 °C, noch vorteilhafter nicht tiefer als 17 °C, noch vorteilhafter nicht tiefer als 19 °C, noch vorteilhafter nicht tiefer als 21 °C, noch vorteilhafter nicht tiefer als 23 °C, noch vorteilhafter nicht tiefer als 25 °C, und noch vorteilhafter nicht tiefer als 27 °C ist.

In einer vorteilhaften Ausführung fliesst ein flüssiges Medium durch den Füllkeil 20, um die gewünschte Temperatur des Füllkeils 20 zu erhalten. In einer vorteilhaften Ausführung ist die Temperatur dieses flüssigen Mediums regelbar.

Insbesondere ist kann dieses flüssige Medium Wasser sein oder beinhalten.

### Temperatur der Formwalzen

Die Temperatur der Formwalzen beeinflusst ebenfalls die Temperatur des Bandes 29 während der Verkapselung und damit auch die Temperatur der Kapselhülle nach der Verkapselung, die vorteilhaft möglichst tief sein soll. Ausserdem wird durch eine tiefe Temperatur der Formwalzen die Entformung der frischen Kapseln 22 erleichtert, insbesondere wird das Kleben der frischen Kapseln 22 an der Formwalze 21 reduziert oder verhindert.

In einer vorteilhaften Ausführung liegt die Temperatur der Formwalzen, gemessen am Umfang mit einem IR-Thermometer, bei höchstens 45 °C, noch vorteilhafter bei höchstens 40 °C, noch vorteilhafter bei höchstens 35 °C, noch vorteilhafter bei höchstens 30 °C, noch vorteilhafter bei höchstens 26 °C, noch vorteilhafter bei höchstens 23 °C, und noch vorteilhafter bei höchstens 20 °C.

In einer vorteilhaften Ausführung ist die Temperatur der Formwalzen nicht tiefer als der Taupunkt der Atmosphäre im Bereich der Verkapselung. Insbesondere ist die Temperatur der Formwalzen vorteilhaft nicht tiefer als 0 °C, noch vorteilhafter nicht tiefer als 5 °C, noch vorteilhafter nicht tiefer als 8 °C, noch vorteilhafter nicht tiefer als 11 °C, und noch vorteilhafter nicht tiefer als 13 °C.

Die Temperatur auf dem Umfang der Formwalzen sollte vorteilhaft über die Länge der Formwalzen 21 einigermassen gleichmässig sein. Vorteilhaft sollen die Temperaturen hier in einem Variationsbereich von nicht mehr als 7 °C, noch vorteilhafter nicht mehr als 5 °C, noch vorteilhafter nicht mehr als 4 °C, noch vorteilhafter nicht mehr als 3 °C, und noch vorteilhafter nicht mehr als 2 °C differieren.

In einer vorteilhaften Ausführung wird die gewünschte Temperatur der Formwalzen 21 mit einem Luftstrom eingestellt, der auf die Formwalzen 21 geblasen wird. Die gewünschte Temperatur der Formwalzen 21 wird insbesondere durch eine geeignete Kombination der Temperatur des Luftstroms und der Menge des Luftstroms (m³/h) erhalten.

In einer vorteilhaften Ausführung ist die Temperatur des Luftstromes regelbar. **In** einer weiteren vorteilhaften Ausführung ist die Menge des Luftstromes regelbar. Am vorteilhaftesten sind sowohl Temperatur als auch Menge des Luftstromes regelbar.

Der Luftstrom wird einerseits auf die linke, andererseits auf die rechte Formwalze 21 gerichtet und so gestaltet, dass die Länge der Formwalzen 21 gleichmässig temperiert werden.

Der Luftstrom hat bei genügender Intensität, die sich durch die Anforderung der Kühlwirkung ergibt, auch eine reinigende Wirkung auf die Formwalze 21, sodass ein solcher Luftstrom vorteilhaft hier auch für gleichbleibende Verhältnisse sorgt.

In einer vorteilhaften Ausführung hat der Luftstrom zur Kühlung der Formwalzen 21 eine Temperatur von weniger als 35 °C, noch vorteilhafter weniger als 30 °C, noch vorteilhafter weniger als 25 °C, noch vorteilhafter weniger als 20 °C, und noch vorteilhafter weniger als 17 °C.

Die Temperierung der Formwalzen 21 kann in einer weiteren vorteilhaften Ausführung dadurch erhalten werden, dass die Formwalzen 21 von einem flüssigen Medium temperiert werden.

Dieses flüssige Medium kann die Formwalzen 21 in einer vorteilhaften Ausführung direkt durchfliessen oder in einer weiteren vorteilhaften Ausführung die zylinderförmige Basis oder Halterung, auf welche die Formwalzen 21 montiert bzw. aufgesteckt werden, wobei dann die Temperatur durch Wärmeleitung von der innen sich befindenden Halterung auf die Formwalzen 21 übertragen wird.

Das flüssige Medium ist in einer vorteilhaften Ausführung Wasser.

In einer weiteren vorteilhaften Ausführung werden die beiden Möglichkeiten der Temperierung durch einen Luftstrom und durch ein flüssiges Medium kombiniert.

### Kühlung der Formwalzen

Vorteilhaft ist im Fall der Flüssigkühlung der Halterung der Formwalzen 21, dass an den Formwalzen 21 selbst, die nicht für eine Temperierung durch ein flüssiges Medium ausgelegt sind, keine Änderungen vorgenommen werden müssen und nach wie vor für verschiedene Kapseltypen ausgetauscht werden können. Nachteilig ist, dass ein Wärmetransport von der Formwalze 21 zur Halterung stattfinden muss und die Distanz vom Inneren, wo sich die Halterung befindet, zum Mantel der Formwalzen 21, dessen Temperatur geregelt werden soll, relativ weit ist.

Werden die Formwalzen 21 direkt von dem flüssigen Medium durchflossen, sind konstruktive Änderungen an den Formwalzen 21 notwendig, da diese nicht ohne weiteres für eine Kühlung durch ein flüssiges Medium geeignet sind.

Formwalzen 21 sind Form- und Stanzwerkzeuge. Auf dem Umfang befinden sich dicht angeordnet Kavitäten, sogenannte Pockets, welche die Form der Weichkapseln vorgeben und um jede Form befindet sich ein Steg, der die Kapsel ausstanzt. Die Kavitäten haben am Boden der Kavitäten jeweils im Zentrum ein Loch, das zur Achse der Formwalze 21 gerichtet ist, und durch welches während der Kapselbildung die Luft aus dem Inneren der Kavität entweichen kann. Durch diese Vorgaben ist es nicht möglich, in einer geraden Linie parallel zur Achse der Formwalze 21 eine Bohrung anzulegen, durch die das flüssige Medium geführt werden kann, ohne dass dieser Kanal durch die genannten Löcher unterbrochen wird. Dazu gibt es verschiedene Lösungsmöglichkeiten.

In einer vorteilhaften Ausführung werden parallel zur Achse der Formwalze 21 Bohrungen angelegt, durch die Röhren gelegt werden. In einer weiteren vorteilhaften Ausführung werden die Entlüftungslöcher nicht zentral im Boden der Kavitäten angelegt, sondern so seitwärts verschoben, dass eine durchgehende geschlossene Bohrung möglich ist. In einer weiteren vorteilhaften Ausführung werden die beiden Möglichkeiten kombiniert.

### Temperatur der frischen Kapseln

Eine tiefere Temperatur der frischen Kapseln 22 bedeutet eine höhere Stabilität dieser Kapseln, was im Hinblick auf die weiteren Verfahrensschritte und schlussendlich für die Qualität der Kapseln ein wichtiger Aspekt ist. Zu warme Kapseln können z.B. einem nachfolgenden Tumbler, wo eine mechanische Belastung stattfindet, beschädigt werden, so dass die Naht sich öffnet oder sich die Qualität der Naht reduziert. Diese Problematik ist bei allen vegetarischen Weichkapseln vorhanden, weshalb diese sehr sorgsam behandelt werden müssen, bis sie durch das Trocknen stabiler und belastbarer werden.

Die Temperatur der Weichkapsel Hülle, gemessen unmittelbar nach der Verkapselung mit einem IR-Thermometer, liegt darum in einer vorteilhaften Ausführung bei nicht mehr als 50 °C, noch vorteilhafter bei nicht mehr als 45 °C, noch vorteilhafter bei nicht mehr als 40 °C, noch vorteilhafter bei nicht mehr als 35 °C, noch vorteilhafter bei nicht mehr als 32 °C, noch vorteilhafter bei nicht mehr als 29 °C, noch vorteilhafter bei nicht mehr als 27 °C, und noch vorteilhafter bei nicht mehr als 25 °C.

Die Temperatur des Inneren der Weichkapsel, gemessen unmittelbar nach der Verkapselung mit einem drahtförmigen Thermoelement, das durch die Hülle in die gefüllte Weichkapsel gesteckt wird, liegt darum ein einer vorteilhaften Ausführung bei nicht mehr als 50 °C, noch vorteilhafter bei nicht mehr als 45 °C, noch vorteilhafter bei nicht mehr als 40 °C, noch vorteilhafter bei nicht mehr als 35 °C, noch vorteilhafter bei nicht mehr als 32 °C, noch vorteilhafter bei nicht mehr als 29 °C noch vorteilhafter bei nicht mehr als 27 °C, und noch vorteilhafter bei nicht mehr als 25 °C.

### Entformung der Kapseln

Nachdem Weichkapseln durch die Formwalzen 21 aus dem zusammengeführten doppelten Band ausgestanzt worden sind, fällt ein Teil der Weichkapseln direkt nach unten, und ein Teil der Weichkapseln bleibt an der Formwalze 21 hängen und wird durch rotierende Walzen abgebürstet. Ein dritter Teil der Weichkapseln bleibt im Netz 23 hängen und wird durch die Abstreifrollen 24 von dem vom doppelten Band übrig gebliebenen Netz 23 separiert, was aber nicht immer vollständig gelingt. Besonders bei grossen Weichkapseln kann das zu gelegentlichen Störungen des Prozesses führen, wenn Weichkapseln von den Abstreifrollen 24 eingeklemmt werden, insbesondere kann das zu einem Unterbruch des Prozesses führen.

In einer vorteilhaften Ausführung wird daher eine zusätzliche Vorrichtung als Entformungshilfe eingesetzt, um die noch im Netz 23 hängenden Weichkapseln davon zu lösen, bevor sie die Abstreifrollen 24 erreichen.

In einer vorteilhaften Ausführung umfasst die Entformungshilfe regelmässige Stösse von Druckluft, um die im Netz 23 noch hängenden Weichkapseln daraus wegzublasen, wobei die Stösse von Druckluft seitwärts auf das vertikal nach unten sich bewegende Band 29 appliziert werden, wobei vorteilhaft die Stösse von Druckluft von beiden Seiten appliziert werden, zeitlich versetzt.

In einer weiteren vorteilhaften Ausführung umfasst die Entformungshilfe regelmässige mechanische Stösse seitwärts auf das Netz 23, um die im Netz 23 noch hängenden Weichkapseln daraus wegzustossen. Die Entformungshilfe führt diese Stösse in vergleichbarer Art aus, wie wenn man mit der Hand von der Seite einen kleinen kurzen, schnellen Klapps gibt. Vorteilhaft werden solche mechanischen Stösse von beiden Seiten appliziert, zeitlich versetzt. Die mechanischen Stösse werden beispielsweise durch eine horizontale Translation eines Stossgebers, durch eine mitlaufende Kulisse oder durch eine exzentrische rotierende Vorrichtung erreicht.

In einer anderen, gegenüber dem Stand der Technik abweichenden und besonders vorteilhaften Ausführungsform wird eine Ablöserolle 39 (Figur 7) vorgesehen, über die das Netz geführt wird, wobei das Netz dabei die Fortbewegungsrichtung ändert und über die Rolle geführt und leicht gespannt ist. Der Umlenkwinkel α beträgt vorteilhafterweise zwischen ca. 2 bis 60°, besonders vorteilhaft ca. 10 bis 50° bei einem Rollendurchmesser von ca. 30 bis 120 mm.

Durch die resultierende transversale bzw. radiale Kraftkomponente werden verbleibende Weichkapseln aus dem Netz herausgedrückt, ohne dass auf die noch temperaturweichen Weichkapseln zu grosse Druck- oder Rückhaltkräfte ausgeübt werden.

Vorteilhaft kann diese Ablösung durch eine auf der von der Rolle abgewandten Seite des Netzes angeordnete rotierende Bürste unterstützt werden.

### Unterstützung der Zugrollen

Eine weitere Prozessstörung, die zum Produktionsunterbruch führen kann, findet bei den Zugrollen statt, welche das Netz 23 sehr stark in die Länge ziehen, wodurch der grösste Teil der Kapseln aus dem Netz 23 fällt. Bei dieser Störung wickelt sich das Netz 23 um die Zugrollen, womit sie ihre Funktionalität verlieren.

In einer vorteilhaften Ausführung werden die Zugrollen kontinuierlich oder regelmässig beölt.

In einer weiteren vorteilhaften Ausführung werden den zwei Zugrollen zwei zusätzliche Zugrollen nachgeschaltet, die das unter den zwei Zugrollen herauskommende Netz 23, das sonst nur von der Gravitation nach unten gezogen wird, zusätzlich nach unten ziehen, vorteilhaft haben sie eine geringere Oberflächengeschwindigkeit als die zwei oberen Zugrollen. Die zusätzlichen, nachgeschalteten Zugrollen können beispielsweise eine glatte Oberfläche haben, oder eine Oberflächenstruktur, die das Netz 23 etwas fassen kann, wie bspw. Riffelwalzen, sie können in Metall oder Kunststoff gefertigt sein und gegebenenfalls auch eine Beschichtung aufweisen.

### Verfahrensschritte nach der Verkapselung

Für die auf die Verkapselung folgenden Verfahrensschritte bis zu den fertigen verpackten Weichkapseln können grundsätzlich alle im Stand der Technik zu Gelatine-Weichkapseln wie auch zu vegetarischen Weichkapseln beschriebenen und gebräuchlichen Verfahrensschritte eingesetzt und auch kombiniert werden.

Im Folgenden werden vorteilhafte Verfahrensvarianten für diese Verfahrensschritte beschrieben, wobei Vorteile erhalten werden können, insbesondere durch Kühlung und durch mechanisch schonende Verfahren.

### Vorkonditionierung durch Kühlung der Weichkapseln nach der Verkapselung

### Variante A

Durch ein kurzes Förderband 26 werden die Weichkapseln 22 nach der Verkapselung aus dem Verkapselungsbereich kontinuierlich wegtransportiert und dann von einem vergleichsweise langen Förderband 27 kontinuierlich zu einem Gebläse gefördert, mit dem die Weichkapseln durch einen Förderkanal 5, insbesondere durch einen rohrförmigen Förderkanal 5, in eine Tumblervorrichtung geblasen werden.

Der Fachmann erkennt, dass in geeigneter Anordnung auch zwei (oder mehr) beidseitig des Netzes bzw. unter den Abstreifrollen 24 angeordnete Förderbänder 26 vorgesehen werden können.

Während dieser Förderung kann eine Vorkonditionierung der Weichkapseln stattfinden.

In einer vorteilhaften Ausführung werden die Weichkapseln auf dem langen Förderband 27 gekühlt, insbesondere werden sie mit einem Luftstrom gekühlt.

In besonderen Ausführungsformen werden im Bereich des Förderbandes 27 ergänzend oder alternativ begleitende Kühlelemente 38.2 angeordnet. Diese können auch als das Förderband umgreifender (isolierter) Kühlkanal ausgebildet sein.

Um Feuchtigkeitskondensationen zu minimieren, können die begleitenden Kühlelemente 38.2 eine degressive Temperaturkurve entlang der Wegförderung der Weichkapseln haben.

In einer weiteren vorteilhaften Ausführung werden die Weichkapseln in dem Kanal 5, der vom Gebläse zur Tumblervorrichtung 7 führt, gekühlt. Da in diesem Kanal eine hohe Strömungsgeschwindigkeit vorhanden ist, ist die Kühlung durch die strömende Luft effektiv. Die Kühlung ist umso effektiver, je länger die Aufenthaltszeit im Kanal ist und je kälter die Luft ist.

In einer vorteilhaften Ausführung beträgt die Länge des Kanals mehr als 1 m, noch vorteilhafter mehr als 1.5 m, noch vorteilhafter mehr als 2 m, noch vorteilhafter mehr als 3 m, noch vorteilhafter mehr als 4 m, und noch vorteilhafter mehr als 5 m.

Auch wenn der Tumbler in kurzer Distanz zum Gebläse lokalisiert ist, kann eine grössere Länge für den Kanal erreicht werden, beispielsweise mit einem als Spirale ausgeführten Förderkanal 5.

Die Luft, welche die Kapseln durch den Kanal transportiert hat in einer vorteilhaften Ausführung eine Temperatur von nicht mehr als 28 °C, noch vorteilhafter nicht mehr als 24 °C, noch vorteilhafter nicht mehr als 20 °C, noch vorteilhafter nicht mehr als 16 °C, noch vorteilhafter nicht mehr als 13 °C, und noch vorteilhafter nicht mehr als 10 °C.

### Variante B: Kühlung in einem Tumbler

Alternativ oder zusätzlich zur Variante A können die Weichkapseln ein einem Tumbler gekühlt werden.

In einer vorteilhaften Ausführung zur Kühlung werden die frischen Weichkapseln 22 einer Tumblervorrichtung zugeführt, insbesondere indem sie mit einem Gebläse über den Förderkanal in diese Tumblervorrichtung geblasen werden, wo sie bewegt und mit der Kühlluft gekühlt werden.

Diese Kühlluft, wird in einer vorteilhaften Ausführung mit einer Temperatur von nicht mehr als 28 °C, noch vorteilhafter nicht mehr als 24 °C, noch vorteilhafter nicht mehr als 20 °C, noch vorteilhafter nicht mehr als 16 °C, noch vorteilhafter nicht mehr als 13 °C, und noch vorteilhafter nicht mehr als 10 °C den zu kühlenden Kapseln zugeführt.

Die Tumblervorrichtung weist mindestens einen Tumbler auf. Typischerweise werden zwei in Serie geschaltete Tumbler eingesetzt. Es sind aber auch drei oder mehr in Serie geschaltete Tumbler möglich.

Tumbler wie sie typischerweise für Weichkapseln verwendet werden, weisen einen Zylinder auf, dessen Achse, um die sich der Zylinder dreht, horizontal liegt. Die Kapseln befinden sich in diesem Zylinder und werden durch die Drehung des Zylinders bewegt und vom Eingang zum Ausgang gefördert, sodass eine Aufenthaltszeit entsteht, die über die Drehgeschwindigkeit und konstruktive Massnahmen definiert werden kann.

Bei herkömmlichen Tumblern, wie sie für Gelatine-Kapseln eingesetzt werden, sind auf der Innenseite Stege angebracht, sodass bei der Drehung der Tumbler Kapseln in grösseren Mengen hochgehoben werden und wieder herunterfallen. Dabei wirken grössere Kräfte auf die Kapseln ein, welche bei vegetarischen Weichkapseln den noch empfindlichen Weichkapselhüllen schaden können, sodass es zu geschwächten Nähten oder sogar zu Lecks in den Schweissnähten kommen kann. Ausserdem werden Tücher in die Tumbler gegeben, woran die Kapseln reiben und somit entölt werden. Mehrere Tumbler können in Serie zu einer gewünschten Gesamtlänge miteinander kombiniert werden.

In einer vorteilhaften Ausführung werden für die Kühlung der Weichkapseln Tumbler verwendet, die innen entweder keine Stege haben oder dann solche mit reduzierter Höhe und gerundeten Kanten, sodass die Weichkapseln darin nicht unnötig stark mechanisch belastet werden.

Insbesondere ist vorteilhaft, dass sich die Kapseln in dem Tumbler vorteilhaft rollend bewegen und nicht oder nur geringfügig fallen.

Ausserdem werden vorteilhaft keine Tücher zugegeben, weil die Entölung zum Verkleben der Kapseln führen könnte, wodurch es zu Agglomeraten kommen kann, die einerseits schlecht gekühlt werden können, und andererseits zu unerwünschter mechanischer Beanspruchung der Weichkapseln führen.

Nach der Kühlung (Variante B) weisen die Weichkapseln sowohl an der Hülle wie auch innen im Füllgut 19 eine Temperatur von nicht mehr als 35 °C auf, noch vorteilhafter von nicht mehr als 30 °C, noch vorteilhafter von nicht mehr als 26 °C, noch vorteilhafter von nicht mehr als 23 °C, noch vorteilhafter von nicht mehr als 21 °C, und noch vorteilhafter von nicht mehr als 19 °C.

Die Temperatur der Hülle wird mit einem IR-Thermometer gemessen, die Temperatur des Füllguts 19 innen mit einem drahtförmigen Thermoelement, wobei der Draht mit der Messstelle durch die Hülle ins Füllgut 19 gesteckt wird.

### Variante B: Kühlung in einem Fluidbett

In einem Fluidbett werden die zu fluidisierenden Güter durch einen Luftstrom in der Schwebe gehalten und so bei minimaler mechanischer Beanspruchung sehr effektiv von Luft umströmt.

In einer vorteilhaften Ausführung wird die Kühlung der Weichkapseln mit einem Fluidbett durchgeführt, insbesondere mit einem kontinuierlich arbeiteten Fluidbett, wobei die zu kühlenden Kapseln sehr schonend gekühlt und bereits auch etwas vorgetrocknet werden.

### Konditionierung der Weichkapseln

Bei der Konditionierung 7 der Weichkapseln werden diese in einer geeigneten Atmosphäre mit definierter Temperatur, relativer Luftfeuchtigkeit und Konvektion auf einen gewünschten Wassergehalt bzw. auf eine gewünschte Wasseraktivität getrocknet. Die anfänglich weichen Kapseln werden dabei deutlich fester, in welchem Zustand sie dann auch ausgeliefert werden.

Die Konditionierung 7 kann bei einer hinsichtlich der Parameter der Atmosphäre konstanten Atmosphäre erfolgen oder die Parameter der Atmosphäre können einen Verlauf aufweisen.

### Konditionierung der Weichkapseln in einer konstanten Atmosphäre

In einer vorteilhaften Ausführung werden die Weichkapseln bei einer mehr oder weniger konstanten Atmosphäre konditioniert, wobei vorteilhaft die nachfolgenden Bedingungen für die Parameter der Atmosphäre gelten. Dabei wird eine hinreichende Konvektion der Atmosphäre erzeugt, damit diese im Konditionierungsraum in etwa gleichbleibend ist und die Weichkapseln von bewegter Luft umströmt werden.

In einer vorteilhaften Ausführung liegt die relative Luftfeuchtigkeit bei nicht mehr als 40%, noch vorteilhafter bei nicht mehr 37%, noch vorteilhafter bei nicht mehr als 35%, und noch vorteilhafter bei nicht mehr als 33%.

In einer vorteilhaften Ausführung liegt die relative Luftfeuchtigkeit bei nicht weniger als 13%, noch vorteilhafter bei nicht weniger als 15%, noch vorteilhafter bei nicht weniger als 17%, und noch vorteilhafter bei weniger als 19%.

In einer vorteilhaften Ausführung liegt die Temperatur bei nicht mehr als 30 °C, noch vorteilhafter bei nicht mehr als 27 °C, noch vorteilhafter bei nicht mehr als 25 °C, noch vorteilhafter bei nicht mehr als 23 °C, und noch vorteilhafter bei nicht mehr als 22 °C.

In einer vorteilhaften Ausführung liegt die Temperatur bei der Konditionierung 7 bei nicht weniger als 10 °C, noch vorteilhafter bei nicht weniger als 13 °C, noch vorteilhafter bei nicht weniger als 15 °C, noch vorteilhafter bei nicht weniger als 17 °C, und noch vorteilhafter bei nicht weniger als 18 °C.

In einer vorteilhaften Ausführung werden die Weichkapseln auf Tabletts gelagert konditioniert.

In einer vorteilhaften Ausführung werden die Weichkapseln in Tumblern konditioniert, wobei die Kapseln bewegt werden, vorteilhaft in einer schonenden Art und Weise wie oben bei der Kühlung (Variante B) der frischen Kapseln 22 beschreiben, wobei insbesondere ein Fallen der Kapseln verhindert wird.

In einer vorteilhaften Ausführung werden die Weichkapseln in den Tumblern während der Konditionierung 7 entölt, indem den Tumblern dazu Tücher zugegeben werden, vorteilhaft nach einigen Stunden der Konditionierung 7, nachdem die Kapseln eine grössere Festigkeit erlangt haben.

In einer vorteilhaften Ausführung wird keine Entölung der Kapseln durchgeführt. In diesem Fall wird die Aussenbeölung so bemessen, dass die Weichkapseln an der Oberfläche kein überschüssiges Öl aufweisen.

### Konditionierung der Weichkapseln bei einer variablen Atmosphäre

In einer weiteren vorteilhaften Ausführung werden die Weichkapseln in einer Atmosphäre konditioniert, bei die Parameter der Atmosphäre über den Lauf der Zeit einen Verlauf aufweisen. Der Verlauf wird vorteilhaft so programmiert, dass die relative Luftfeuchtigkeit im Verlauf der Konditionierung 7 abnimmt und erst in der Schlussphase die oben angegebenen Bedingungen für eine konstante Atmosphäre erreicht. Damit kann die Konditionierung 7 beschleunigt ablaufen. Ebenso kann die Temperatur auf einen gewünschten Verlauf angepasst werden.

### Giessmasse

### Rezeptur der Giessmasse

Bei allen Verfahren zur Herstellung von vegetarischen Weichkapseln besteht die Herausforderung, das Rotary-Die-Verfahren mit einem gegenüber Gelatine deutlich weicheren Band 29 durchzuführen, was zu erheblichen Schwierigkeiten führt. Die vegetarischen Bänder sind teilweise so weich, dass sie unter dem Eigengewicht bereits durchhängen und gedehnt werden. Der Abzug des Bandes 29 von der Giesstrommel 13 und die Bandführung bis zur Verkapselung ist umso schwieriger, je höher die Bandgeschwindigkeit und damit die Produktionsgeschwindigkeit ist.

Die Robustheit des Prozesses und die Qualität der Produkte, insbesondere bei hohen Produktionsgeschwindigkeiten, nehmen daher mit zunehmender Festigkeit des Bandes 29 zu.

Andererseits hat die Rezeptur der Geissmasse damit des Bandes 29 auch einen Einfluss auf das Verschweissverhalten bei der Verkapselung und damit auf die Qualität der Schweissnaht.

Es wurde gefunden, dass gegenüber dem Stand der Technik beschrieben in WO 2010/100196 A1 noch eine erstaunlich grosse Verbesserung bei der Festigkeit des Bandes erreicht werden konnte.

Insbesondere konnte der E-Modul des Bandes in einem bedeutenden Umfang verbessert werden, wodurch der Prozess deutlich vereinfacht und beschleunigt werden konnte, wobei gleichzeitig auch eine optimale Verschweissung erreicht werden konnte.

Dies wurde einerseits durch eine besonders vorteilhafte Auswahl der im genannten Stand der Technik beschriebenen Rezepturbereiche der Giessmasse 11 betreffend die Stärke und deren Zustand, sowie betreffend Weichmacher und Wasser erhalten, und andererseits durch den Einsatz von einem besonders vorteilhaften Anteil an Verdickungsmittel und insbesondere mit der Auswahl von besonders vorteilhaften Verdickungsmitteln.

In einer vorteilhaften Ausführung weist die Giessmasse 20 - 45 Gew.-% Wasser auf und die trockene Giessmasse weist folgende Komponenten auf:
a) 40 - 80 Gew.-% Stärke, wobei mindestens 80% der Stärke in Form von Partikeln von granulärer Stärke vorliegt;
b) 20 - 65 Gew.-% Weichmacher;
c) 0 - 7 Gew.-% Verdickungsmittel;
d) optional maximal 10 Gew.-% gelöste Stärke; und
e) optional übliche Zusatz- und Hilfsstoffe.

Unter dem Wasser wird hier das totale Wasser verstanden, dass sich aus dem zugegebenen Wasser und dem in den Komponenten als Feuchtigkeit enthaltenen Wasser ergibt. Die Anteile der Komponenten beziehen sich entsprechend auf die wasserfreien Komponenten.

### Stärke

Grundsätzlich können hinsichtlich des Ursprungs und Aufbereitung beliebige Stärken oder Mischungen davon eingesetzt werden. Sie können zum Beispiel im nativen Zustand, wie auch im physikalisch und/oder chemisch oder enzymatisch modifizierten Zustand eingesetzt werden.

In einer vorteilhaften Ausführung wird die Stärke im nativen, d.h. weder chemisch noch physikalisch modifizierten Zustand eingesetzt. Hiermit können gute Eigenschaften bei tiefen Kosten erhalten werden. Ausserdem sind nicht chemisch modifizierte Stärken am Markt vorteilhaft, weil sie natürlich sind, keine E-Nummer haben und geeignet sind für Clean Labels, d.h. in der Liste der Zusammensetzung kann die Stärke als «Stärke» gelistet werden. Darüber hinaus sind native Stärke in Bio-Qualität erhältlich, während nach einer chemischen Modifikation keine Bio-Qualität mehr möglich ist.

Hinsichtlich des Ursprungs sind Wurzelstärken wie beispielweise Kartoffelstärken oder Tapiokastärken vorteilhaft, da diese im Vergleich zu Stärken anderer Herkunft niedrigere Gelatinisierungstemperaturen aufweisen und die Verfestigung bzw. Gelierung der Giessmasse 11 zu Filmen 28 zur Weichkapselherstellung daher bereits bei niedrigen Temperaturen möglich ist.

Besonders vorteilhaft ist Tapiokastärke. Tapiokastärke ist farblos, geschmacklos, weist eine sehr gute Transparenz auf und es sind von Tapiokastärken noch keine genetisch modifizierten Varianten bekannt. Besonders vorteilhaft wird native Tapiokastärke eingesetzt.

In einer weiteren vorteilhaften Ausführungsform werden chemisch modifizierte, substituierte Stärken wie Stärkeester und Stärkeether eingesetzt wie beispielsweise hydroxypropylierte oder acetylierte Stärken. Diese Modifikationen führen zu besonders hoher Transparenz und hohem Dehnvermögen des Films 28 beziehungsweise des Bands 29. Alternativ können oxidierte Stärken eingesetzt werden.

In einer weiteren vorteilhaften Ausführungsform werden vernetzte Stärken eingesetzt, insbesondere vernetzte Stärkeester bzw. vernetzte Stärkeether, beispielsweise Stärkephosphate und Stärkeadipate.

Durch die Erhöhung des Molekulargewichtes, welche mit der Vernetzung einhergeht, werden verbesserte mechanische Eigenschaften erhalten und werden die Stärkekörner als Einheiten auch mechanisch stabilisiert, was für das Verfahren besonders vorteilhaft ist, da damit der Beitrag der Stärkepartikel zu den mechanischen Eigenschaften des frischen Films 28, des Bandes 29, der frischen Weichkapsel 22 und der getrockneten Weichkapsel gesteigert werden kann. Bei hochvernetzten Stärken bildet das destrukturierte Stärkekorn faktisch ein Molekül von sehr grossem Molekulargewicht und weist eine besonders hohe Stabilität auf.

In einer weiteren vorteilhaften Ausführungsform wird substituierte Tapiokastärke eingesetzt, insbesondere vernetzte substituierte Tapiokastärke wie beispielsweise hydroxypropyliertes Stärkephosphat.

Das vorteilhafte Gewichtsmittel der Molekulargewichtsverteilung M_{w}1 der verwendeten Stärke liegt mindestens bei 500.000 g/mol, noch vorteilhafter bei mindestens 1.000.000 g/mol, noch vorteilhafter bei mindestens 2.500.000 g/mol, noch vorteilhafter bei mindestens 3.000.000 g/mol, noch vorteilhafter bei mindestens 4.000.000 g/mol, noch vorteilhafter bei mindestens 5.000.000 g/mol, noch vorteilhafter bei mindestens 7.000.000 g/mol, und noch vorteilhafter bei mindestens 10.000.000 g/mol.

Weiterhin sind Wachs-Stärken (Waxy-Stärken) vorteilhaft, insbesondere vernetzte und/oder substituierte Waxy-Stärken. Waxy-Stärken sind betreffend die Transparenz vorteilhaft.

Die im Folgenden genannten vorteilhaften Anteile an Stärke beziehen sich jeweils auf die trockene Stärke, die auf die trockene Giessmasse bezogen wird.

In einer vorteilhaften Ausführungsform der Erfindung liegt die untere Grenze des Stärkeanteils in der trockenen Giessmasse in Gew.-% bei > 40, noch vorteilhafter bei > 45, noch vorteilhafter bei > 50, noch vorteilhafter bei > 53, noch vorteilhafter bei > 55, noch vorteilhafter bei > 56, und noch vorteilhafter bei > 57.

In einer vorteilhaften Ausführungsform der Erfindung liegt die obere Grenze des Stärkeanteils in der trockenen Giessmasse in Gew.-% bei < 80, noch vorteilhafter bei < 75, noch vorteilhafter bei < 72, noch vorteilhafter bei < 70, noch vorteilhafter bei < 68, noch vorteilhafter bei < 67, noch vorteilhafter bei < 66, noch vorteilhafter bei < 65, und noch vorteilhafter bei < 65.

Ist in einer vorteilhaften Ausführung der Weichmacher im Wesentlichen Glycerin, liegt in einer vorteilhaften Ausführung die untere Grenze des Stärkeanteils in der trockenen Giessmasse in Gew.-% bei > 50, noch vorteilhafter bei > 55, noch vorteilhafter bei > 56, noch vorteilhafter bei > 57, noch vorteilhafter bei > 58, noch vorteilhafter bei > 59, und noch vorteilhafter bei > 60.

Ist in einer vorteilhaften Ausführung der Weichmacher im Wesentlichen Glycerin, liegt in einer vorteilhaften Ausführung die obere Grenze des Stärkeanteils in der trockenen Giessmasse in Gew.-% bei < 75, noch vorteilhafter bei < 70, noch vorteilhafter bei < 69, noch vorteilhafter bei < 68, noch vorteilhafter bei < 67, noch vorteilhafter bei < 66, noch vorteilhafter bei < 65, und noch vorteilhafter bei < 64.

### Granuläre Stärke

Die Stärke wird in Form von granulärer Stärke eingesetzt. Diese granuläre Stärke ist ein Pulver von Stärkepartikeln, wobei diese Partikel in ihrer Form den ursprünglichen, nativen Stärkekörnern entsprechen, wie sie in den Pflanzen entstanden sind, oder es handelt sich um Agglomerate davon. Typische Grössen der nativen Stärkekörner sind: 5 - 100 µm bei Kartoffelstärke, 5 - 30 µm bei Maisstärke, 1 - 45 µm bei Weizenstärke, 4 - 35 µm bei Tapiokastärke, und 1 - 30 µm bei Reisstärke. Als granuläre Stärke können auch Mischungen von verschiedenen granulären Stärken eingesetzt werden.

Native Stärkekörner sind teilkristallin und können unter gekreuzten Polarisatoren im Mikroskop gut identifiziert und charakterisiert werden (vgl. Ausführungen zu Figur 3(a)). Die in einem nativen Stärkekorn enthaltenen Kristallite sind doppelbrechend und erzeugen dabei eine als «Malteserkreuz» bekannte, charakteristische Struktur womit der Zustand der Stärkekörner identifiziert werden kann.

Mit dieser teilkristallinen Struktur sind die Stärkekörner in Wasser und Weichmacher bei Raumtemperatur und oberhalb davon bis zum Bereich der Gelatinisierungstemperatur nicht löslich und nicht quellbar.

Am unteren Ende des Bereichs der Gelatinisierungstemperatur beginnt die Destrukturierung der Stärkekörner, wobei die doppelbrechende Eigenschaft verschwindet. Am oberen Ende dieses Bereichs sind alle Stärkekörner nicht mehr doppelbrechend. Sie liegen dann infolge der Phasentransformation in einer stark gequollenen Form vor, wobei die Teilkristallinität verschwunden ist. Dabei sind die Makromoleküle hydratisiert, d.h. von Wasser-Molekülen umgeben, doch resultiert nicht eine echte Lösung der Stärkemoleküle im Wasser, sondern die ursprünglichen Stärkekörner sind durch die Aufnahme des Wassers auf ein Vielfaches der ursprünglichen Grösse gequollen und bleiben immer noch als eine zusammenhängende Struktur erhalten, die unter dem Mikroskop identifiziert werden kann.

Sind 1 Gew.-% doppelbrechende Stärkekörner in einer wässrigen Flüssigkeit suspendiert, entsteht durch die Gelatinisierung eine massive Zunahme der Viskosität, da die Stärkekörner sehr viel Wasser binden. Bei einem deutlich höheren Anteil von Stärkekörnern wird die wässrige Flüssigkeit in einen elastisch-plastischen Feststoff umgewandelt. Dies ist der Vorgang, durch den bei der vorliegenden Erfindung die viskose und somit formbare Giessmasse 11 auf der geheizten Giesstrommel 13 unter Gelatinisierung innerhalb von wenigen Sekunden in ein Band 29 umgewandelt wird.

In einer vorteilhaften Ausführung sind mindestens 70% der Stärkekörner der granulären Stärke doppelbrechend, noch vorteilhafter mindestens 80%, noch vorteilhafter mindestens 90%, noch vorteilhafter mindestens 95%, und noch vorteilhafter mindestens 97%. Noch vorteilhafter sind im Wesentlichen praktisch alle Stärkekörner noch doppelbrechend.

«Im Wesentlichen» ist so zu verstehen, dass die doppelbrechende Eigenschaft der granulären Stärke gleich ist, wie das bei der ursprünglichen, nativen Stärke typischerweise der Fall ist. Dies bedeutet wiederum, dass die Stärke keine Behandlung erlebt hat, welche die kristalline Struktur in einem messbaren Umfang verändert hat.

Vorteilhaft liegt der Anteil der trockenen granulären Stärke am gesamten, trockenen Stärkeanteil der Giessmasse 11 in Gew.-% bei > 80, noch vorteilhafter bei > 85, noch vorteilhafter bei > 90, noch vorteilhafter bei > 93, noch vorteilhafter bei > 95, noch vorteilhafter bei > 96, noch vorteilhafter bei > 97, und noch vorteilhafter bei > 98.

Das Vorliegen von granulärer Stärke in der Giessmasse 11 ist charakteristisch für die vorliegende Erfindung und einmalig für die Technologie. Bei anderen Giessmassen 11 zur Herstellung von vegetarischen Weichkapseln findet sich hingegen keine granuläre Stärke in der Giessmasse 11, beziehungsweise nur in Kombination mit einem Geliermittel.

Die granuläre Stärke wird bei der Umwandlung der Giessmasse 11 in einen mehr oder weniger festen Film gelatinisiert, wobei die Stärkekörner massiv grösser werden, aber immer noch als Partikel vorliegen und sich in dieser Form im Band 29 und schliesslich in der Weichkapselhülle wiederfinden. Im Lichtmikroskop können die Stärkekörner identifiziert und auch quantitativ mit Wiedergewinnungsverfahren, die weiter unten spezifiziert sind, bestimmt werden.

Da die flüssigen Komponenten bei der Gelatinisierung von den Stärkekörnern vollständig aufgesaugt werden, besteht das Band 29 im Wesentlichen aus dicht aneinander liegenden, miteinander verbundenen, gelatinisierten Stärkekörnern. Die Makromoleküle der Verdickungsmittel befindenden sich zwischen diesen Stärkekörnern.

### Wasser

Wasser ist für die Einstellung der Viskosität der Giessmasse 11 und für die Gelatinisierung der Giessmasse 11 notwendig, bei welcher die Giessmasse 11 zu einem Band 29 transformiert wird.

Je höher der Wassergehalt ist, umso einfacher ist die Herstellung der Giessmasse 11 und das Giessen, umso schneller erfolgt die Verfestigung durch Gelatinisierung, und umso geringer ist die dafür notwendige Temperaturerhöhung. Andererseits jedoch reduziert ein hoher Wassergehalt die Festigkeit des Bandes 29, wodurch die Bandführung erschwert und die Verstreckung des Bandes 29, die minimal gehalten werden soll, erhöht werden muss.

Besonders für höhere Produktionsgeschwindigkeiten ist ein möglichst fester Film erwünscht, was hinsichtlich des Wassergehaltes einen möglichst tiefen Wassergehalt des Bandes 29 und damit auch der Giessmasse 11 bedeutet. Ausserdem wird durch einen tiefen Wassergehalt das spätere Trocknen der Weichkapseln erleichtert, weil weniger Wasser wegzutrocknen ist.

Als Wassergehalt wird die Gesamtmenge an Wasser verstanden, also die Summe des mit der Rezeptur zugegebenen Wasser plus den Wasseranteilen der weiteren Rezepturkomponenten wie beispielsweise dem in der Stärke enthaltenen Wasser, dem im Weichmacher enthaltenen Wasser, dem im Verdickungsmittel enthaltenen Wasser.

Die obere Grenze des Wassergehalts der Giessmasse 11 in Gew.-% beträgt vorteilhaft 45, noch vorteilhafter 42, noch vorteilhafter 40, noch vorteilhafter 39, noch vorteilhafter 38, noch vorteilhafter 37, noch vorteilhafter 36, noch vorteilhafter 35 und noch vorteilhafter 34.

Die untere Grenze des Wassergehalts der Giessmasse 11 in Gew.-% beträgt vorteilhaft 20, noch vorteilhafter 22, noch vorteilhafter 24, noch vorteilhafter 25, noch vorteilhafter 26, noch vorteilhafter 27, noch vorteilhafter 28, noch vorteilhafter 29, und noch vorteilhafter 30.

### Weichmacher

Als Weichmacher kommen grundsätzlich alle aus Stand der Technik bekannten Weichmacher für Stärke in Frage. Die bei Gelatine eingesetzten Weichmacher und Mischungen davon können ebenfalls für die auf Stärke basierten Weichkapseln eingesetzt werden.

Weichmacher können einzeln oder in Mischungen von verschiedenen Weichmachern eingesetzt werden. Vorteilhaft werden Polyole eingesetzt wie beispielsweise Glycerin, Sorbitol, Maltitol, Erythritol, Xylitol, Mannitol, Galactitol, Tagatose, Lactitol, Maltulose, Isomalt, Maltol usw., aber auch verschiedene Zucker wie Sucrose, Maltose, Trehalose, Lactose, Lactulose, Galactose, Fructose usw. sowie Mono- und Oligosaccharide.

Glycerin ist als Weichmacher besonders vorteilhaft. Weiter ist Sorbitol besonders vorteilhaft, insbesondere in Kombination mit Glycerin.

Wasser ist ebenfalls ein Weichmacher für Stärke, wird hier jedoch nicht zu den Weichmachern gerechnet und separat berücksichtigt.

Ein zu tiefer Weichmachergehalt führt zu Versprödung der Weichkapseln bei tiefen Luftfeuchtigkeiten, während ein zu hoher Weichmachergehalt zu schlechten Eigenschaften bei hoher Luftfeuchtigkeit führt, die Weichkapseln werden dann zu weich und sind nicht mehr formstabil.

Die im folgenden genannten Weichmachergehalte beziehen sich jeweils auf den trockenen (also wasserfreien) Weichmacher, der auf die trockene Giessmasse bezogen wird.

Die obere Grenze für den Weichmachergehalt in der trockenen Giessmasse in Gew.-% beträgt vorteilhaft 65, noch vorteilhafter 60, noch vorteilhafter 55, noch vorteilhafter 52, noch vorteilhafter 49, noch vorteilhafter 46, noch vorteilhafter 43, noch vorteilhafter 42, und noch vorteilhafter 41.

Besteht in einer vorteilhaften Ausführung der Weichmacher im Wesentlichen aus Glycerin, beträgt die obere Grenze für den Weichmachergehalt in der Giessmasse in Gew.-% vorteilhaft 50, noch vorteilhafter 46, noch vorteilhafter 45, noch vorteilhafter 42, noch vorteilhafter 41, noch vorteilhafter 40, noch vorteilhafter 39, noch vorteilhafter 38, und noch vorteilhafter 37.

Die untere Grenze für den Weichmachergehalt der Giessmasse in Gew.-% beträgt vorteilhaft 20, noch vorteilhafter 23, noch vorteilhafter 26, noch vorteilhafter 27, noch vorteilhafter 28, noch vorteilhafter 29, noch vorteilhafter 30, noch vorteilhafter 31 und noch vorteilhafter 32.

### Verdickungsmittel

Der Stärke enthaltenden Giessmasse 11 wird vorteilhaft ein Verdickungsmittel zugesetzt, einerseits um die Viskosität der Giessmasse 11 auf einen gewünschten Wert einzustellen, so dass die Partikel der granulären Stärke gleichmässig suspendiert sind und beim Stehenlassen der Giessmasse 11 nicht sedimentieren. Andererseits ist das Verdickungsmittel von Bedeutung, um die Festigkeit des Bandes 29 zu erhöhen, was für einen robusten Prozess mit hoher Qualität der Produkte und dies insbesondere bei hohen Produktionsgeschwindigkeiten von Bedeutung ist.

Als Verdickungsmittel kommen grundsätzlich alle hydrophilen Substanzen und Mischungen davon in Frage, welche die Viskosität erhöhen, insbesondere hydrophile Polymere und vorteilhaft solche aus pflanzlichen Quellen.

Beispiele sind Hydrokolliode und Gummis wie Galactomannane, wie Guar-Gummi oder Johannisbrotkernmehl; Cellulosederivate; Pectine, insbesondere Rhamnogalakturonane und Protopektine; Dextrane; Xanthan; Zymosan; Hydrokolloide aus Meeresalgen, wie Alginate, Agar-Agar, Agarose, Carrageen und Carrageenane; Furcellaran; Hydrokolloide aus Flechten, wie Lichenine und Isolichenine, oder Hydrokolloide als Exsudate aus Hölzern, wie Tragant (Astragalus Gummi), Karaya-Gummi, Gummi arabicum, Kutira-Gummi; Inulin; Latex; Chitin; Chitosan; Gellan; Casein; Collagen, Gelatine. Als Verdickungsmittel wird einer der genannten Stoffe oder eine Kombination davon verstanden.

Gelöste Stärke kann für dieselbe Funktionalität wie die Verdickungsmittel eingesetzt werden, wird aber nicht zu den Verdickungsmitteln gerechnet und separat behandelt.

Einige dieser Verdickungsmittel wie beispielsweise Carrageenan, Gellan und Pectin sind aus dem Stand der Technik auch als Geliermittel bekannt, wobei sie allerdings beim Abkühlen gelieren, und nicht beim Erhitzen, wie dies bei der vorliegenden Erfindung für die Bildung des Bandes 29 aus der Giessmasse 11 charakteristisch ist. Sie leisten bei der Verfestigung der erfindungsgemässen Giessmischung bei der Temperaturerhöhung keinen Beitrag zur Gelierung und werden auch nicht für diesen Zweck eingesetzt.

In den folgenden vorteilhaften Ausführungsformen betreffend Verdickungsmittel und individuell genannten Verdickungsmitteln beziehen sich die genannten Anteile jeweils auf das trockene Verdickungsmittel, das auf die trockene Giessmasse bezogen wird.

In einer vorteilhaften Ausführungsform liegt der maximale Anteil an Verdickungsmittel in der trockenen Giessmasse in Gew.-% bei 7, noch vorteilhafter bei 6, noch vorteilhafter bei 5, noch vorteilhafter bei 4, noch vorteilhafter bei 3,5, noch vorteilhafter bei 3,25, noch vorteilhafter bei 3,00, noch vorteilhafter bei 2,75, und noch vorteilhafter bei 2,50; während der minimale Anteil an Verdickungsmittel in der trockenen Giessmasse in Gew.-% vorteilhaft bei 0,5 liegt, noch vorteilhafter bei 0,6, noch vorteilhafter bei 0,7, noch vorteilhafter bei 0,8, noch vorteilhafter bei 0,9, noch vorteilhafter bei 1,0, noch vorteilhafter bei 1,1, noch vorteilhafter bei 1,2, noch vorteilhafter bei 1,3 und noch vorteilhafter bei 1,4.

In einer anderen vorteilhaften Ausführungsform weist das Verdickungsmittel Guar auf, wobei der maximale Anteil an Guar in der trockenen Giessmasse in Gew.-% bei 7 liegt, noch vorteilhafter bei 6, noch vorteilhafter bei 5, noch vorteilhafter bei 4, noch vorteilhafter bei 3,5, noch vorteilhafter bei 3,25, noch vorteilhafter bei 3,00, noch vorteilhafter bei 2,75, und noch vorteilhafter bei 2,50; während der minimale Anteil an Guar in der trockenen Giessmasse in Gew.-% vorteilhaft bei 0,5 liegt, noch vorteilhafter bei 0,6, noch vorteilhafter bei 0,7, noch vorteilhafter bei 0,8, noch vorteilhafter bei 0,9, noch vorteilhafter bei 1,0, noch vorteilhafter bei 1,1, noch vorteilhafter bei 1,2, noch vorteilhafter bei 1,3 und noch vorteilhafter bei 1,4.

In einer weiteren vorteilhaften Ausführungsform weist das Verdickungsmittel Johannisbrotkernmehl auf, wobei der maximale Anteil an Johannisbrotkernmehl in der trockenen Giessmasse in Gew.-% bei 7 liegt, noch vorteilhafter bei 6, noch vorteilhafter bei 5, noch vorteilhafter bei 4, noch vorteilhafter bei 3,5, noch vorteilhafter bei 3,25, noch vorteilhafter bei 3,00, noch vorteilhafter bei 2,75, und noch vorteilhafter bei 2,50; während der minimale Anteil an Johannisbrotkernmehl in der trockenen Giessmasse in Gew.-% vorteilhaft bei 0,5 liegt, noch vorteilhafter bei 0,6, noch vorteilhafter bei 0,7, noch vorteilhafter bei 0,8, noch vorteilhafter bei 0,9, noch vorteilhafter bei 1,0, noch vorteilhafter bei 1,1, noch vorteilhafter bei 1,2, noch vorteilhafter bei 1,3 und noch vorteilhafter bei 1,4.

In noch einer anderen vorteilhaften Ausführungsform weist das Verdickungsmittel Xanthan auf, wobei der maximale Anteil an Xanthan in der trockenen Giessmasse in Gew.- % bei 7 liegt, noch vorteilhafter bei 6, noch vorteilhafter bei 5, noch vorteilhafter bei 4, noch vorteilhafter bei 3,5, noch vorteilhafter bei 3,25, noch vorteilhafter bei 3,00, noch vorteilhafter bei 2,75, und noch vorteilhafter bei 2,50; während der minimale Anteil an Xanthan in der trockenen Giessmasse in Gew.-% vorteilhaft bei 0,5 liegt, noch vorteilhafter bei 0,6, noch vorteilhafter bei 0,7, noch vorteilhafter bei 0,8, noch vorteilhafter bei 0,9, noch vorteilhafter bei 1,0, noch vorteilhafter bei 1,1, noch vorteilhafter bei 1,2, noch vorteilhafter bei 1,3 und noch vorteilhafter bei 1,4.

In einer vorteilhaften Ausführungsform beträgt der maximale Anteil an Carrageen in der trockenen Giessmasse 11 in Gew.-%, 5, noch vorteilhafter 2, noch vorteilhafter 1, noch vorteilhafter 0,5, noch vorteilhafter 0,25, und noch vorteilhafter 0,1. Noch vorteilhafter liegt dieser Anteil bei 0%.

In einer vorteilhaften Ausführungsform beträgt der maximale Anteil an Gelatine in der trockenen Giessmasse 11 in Gew.-%, 5, noch vorteilhafter 2, noch vorteilhafter 1, noch vorteilhafter 0,5, noch vorteilhafter 0,25, und noch vorteilhafter 0,1. Noch vorteilhafter liegt dieser Anteil bei 0%.

In einer vorteilhaften Ausführungsform beträgt der maximale Anteil an Gellan in der trockenen Giessmasse 11 in Gew.-%, 5, noch vorteilhafter 2, noch vorteilhafter 1, noch vorteilhafter 0,5, noch vorteilhafter 0,25, und noch vorteilhafter 0,1. Noch vorteilhafter liegt dieser Anteil bei 0%

In einer vorteilhaften Ausführungsform beträgt der maximale Anteil an Pektin in der trockenen Giessmasse 11 in Gew.-%, 5, noch vorteilhafter 2, noch vorteilhafter 1, noch vorteilhafter 0,5, noch vorteilhafter 0,25, und noch vorteilhafter 0,1. Noch vorteilhafter liegt dieser Anteil bei 0%.

In einer vorteilhaften Ausführungsform beträgt der maximale Anteil an Cellulosederivaten in der trockenen Giessmasse 11 in Gew.-%, 10, noch vorteilhafter 5, noch vorteilhafter 2,5, noch vorteilhafter 1, noch vorteilhafter 0,5, noch vorteilhafter 0,25, und noch vorteilhafter 0,1. Noch vorteilhafter liegt dieser Anteil bei 0%.

### Gelöste Stärke

In einer vorteilhaften Ausführungsform kann gelöste Stärke ebenso wie die zuvor erwähnten Verdickungsmittel eingesetzt werden, um die Viskosität der Mischung zu erhöhen und um die Festigkeit des Bandes 29 zu erhöhen, ausserdem kann damit die spätere Freisetzung des Inhalts der Weichkapseln in wässrigem Milieu, insbesondere nach dem Verschlucken durch einen Anwender, beschleunigt werden.

Betreffend die gelöste Stärke gelten dieselben Spezifikationen zu geeigneten Stärken und vorteilhaften Typen wie betreffend die Stärke allgemein. Jedoch kann die gelöste Stärke auch ein tieferes Molekulargewicht aufweisen als für die Stärke allgemein vorteilhaft ist. Ausserdem werden für die gelöste Stärke noch vorteilhaft stark retrogradationsstabilisierte Stärken eingesetzt, so dass nach dem Konsum der Weichkapsel durch einen Anwender die Freisetzung des Inhalts der Weichkapsel in wässrigem Medium beschleunigt werden kann.

Die gelöste Stärke unterscheidet sich von der granulären Stärke in ihrem Zustand in der Giessmasse 11, wo die gelöste Stärke in destrukturierter, nicht doppelbrechender, in gelatinisierter Form, d.h. gequollen, hydratisiert oder gelöst vorliegt, während die granuläre Stärke zu diesem Zeitpunkt noch vorwiegend nicht destrukturiert, noch doppelbrechend, nicht gelatinisiert vorliegt. Mit «gelöster Stärke» wird also der Zustand dieser Stärke in der Giessmasse 11 vor der Umwandlung der Giessmasse 11 in das Band 29 bezeichnet, wobei die gelöste Stärke in der Giessmasse 11 ganz oder teilweise in gequollener, hydratisierter oder gelöster Form vorliegt.

In den folgenden vorteilhaften Ausführungsformen betreffend gelöste Stärke beziehen sich die genannten Anteile jeweils auf die trockene gelöste Stärke, die auf die trockene Giessmasse bezogen wird.

In einer vorteilhaften Ausführungsform liegt der maximale Anteil der gelöste Stärke in der trockenen Giessmasse 11, in Gew.-% bei 10, noch vorteilhafter bei 8, noch vorteilhafter bei 7, noch vorteilhafter bei 6, noch vorteilhafter bei 5, und noch vorteilhafter bei 4, während der minimale Anteil der gelösten Stärke in der trockenen Giessmasse 11 in Gew.-% vorteilhaft bei 0,5 liegt, noch vorteilhafter bei 0,7, noch vorteilhafter bei 0,9, noch vorteilhafter bei 1,0, noch vorteilhafter bei 1,1, noch vorteilhafter bei 1,2, noch vorteilhafter bei 1,3, und noch vorteilhafter bei 1,4.

### Weitere Bestandteile (Zusatz- und Hilfsstoffe)

Weitere Bestandteile der Giessmasse können Zusatz- und Hilfsstoffe sein, insbesondere Farbstoffe und Pigmente, Füllstoffe, insbesondere mineralische Füllstoffe wie beispielsweise Talk oder Silikate, oder modifizierende Stoffe wie Polyethlyenglycole oder Zerfallshilfen wie beispielsweise Carbonate oder Hydrogencarbonate, oder Additive wie beispielsweise Konservierungsmittel, Antioxidantien oder Emulgatoren wie beispielsweise Lecithine, Monoglyceride, Diglyceride und Triglyceride von Fettsäuren, Polyglycerinester, Polyethylenester oder Zuckerester.

Grundsätzlich können alle Zusatzstoffe, welche bei Gelatineweichkapselhüllen oder vegetarischen Weichkapselhüllen eingesetzt werden, erfindungsgemäss ebenfalls eingesetzt werden, insbesondere Zusatzstoffe, welche eingesetzt werden um die Weichkapselhülle auf den Inhaltsstoff anzupassen (Formulierungshilfsstoffe).

### Herstellung der Giessmasse

Die Giessmasse 11 wird durch Mischen der folgenden Komponenten erhalten:
a) Stärke, die vorwiegend granuläre Stärke umfasst
b) Weichmacher
c) Wasser
d) Verdickungsmittel
e) gegebenenfalls gelöste Stärke
f) gegebenenfalls übliche Zusatz- und Hilfsstoffe

Die Mischung kann in einer Vielzahl von Mischern 1 erfolgen, wie sie im Stand der Technik in sehr grosser Zahl bekannt sind, wobei insbesondere Mischer 1 eingesetzt werden, die eine gute Mischwirkung aufweisen, d.h. die distributive, verteilende Wirkung ist wesentlich für eine gute Homogenisierung der Komponenten der Giessmasse 11, während eine dispersive, zerteilende Wirkung mit hohen Scherkräften nur in geringem Umfang benötigt wird.

In einer vorteilhaften Ausführung wird das Mischen dazu genutzt, die Mischung aufzuheizen, insbesondere auf eine Temperatur im Bereich von 30 bis 70 °C, vorteilhaft im Bereich von 35 bis 65 °C, noch vorteilhafter im Bereich von 40 bis 60 °C und noch vorteilhafter 45 bis 55 °C.

Indem die Giessmasse 11 bereits bei der Herstellung aufgeheizt wird, wobei insbesondere der Energieeintrag durch die Mischleistung dafür genutzt werden kann, muss später, wenn die Giessmasse 11 gegossen und auf der Giesstrommel 13 aufgeheizt werden soll, weniger aufgeheizt werden, wodurch die dort verlangte Temperatur im Bereich von 100 °C schneller erreicht wird, was insbesondere bei hohen Produktionsgeschwindigkeiten vorteilhaft oder notwendig ist.

### Mischverfahren (Variante A)

In einer vorteilhaften Ausführung wird die Giessmasse 11 in einem Mischgefäss mit einem Rührwerk hergestellt, wobei das Rührwerk für eine vorwiegend distributive Mischung ausgelegt ist.

Das Mischverfahren wird vorteilhaft in folgenden Schritten durchgeführt:
a) Herstellung einer Vormischung 1 durch Einmischen des Verdickungsmittels und gegebenenfalls der gelösten Stärke in den Weichmacher und Homogenisieren
b) Herstellung einer Vormischung 2 durch Einmischen des Wassers in die Vormischung 1 und Homogenisieren
c) Einmischen der Stärke in die Vormischung 2
d) Homogenisieren

Gegebenenfalls werden in mindestens einem der Schritte übliche Zusatz- und Hilfsstoffe noch eingemischt, vorteilhaft in Schritt b), noch vorteilhafter in Schritt a).

In einer vorteilhaften Ausführung wird das Mischverfahren unter Vakuum durchgeführt, vorteilhaft wird in mindestens einem der Schritte c) und d) Vakuum eingesetzt.

In einer vorteilhaften Ausführung wird in Schritt c) die Stärke mit Vakuum in den Mischer 1 eingesogen.

### Mischverfahren (Variante B)

Beim Mischverfahren (Variante B) ist der Mischbehälter zusätzlich mit einem Mischkreislauf zum Einmischen von insbesondere pulverförmigen Komponenten in eine flüssige Phase ausgestattet. Dabei führt eine Leitung vom Mischgefäss zu einer kontinuierlich arbeitenden Pulvermischeinheit, umfassend eine Pumpe, welche den Kreislauf erzeugt, einen Einzug für das Pulver und ein Mischaggregat.

Nach der Pulvermischeinheit wird die Masse zurück in den Mischbehälter geführt. Im Mischbehälter kann eine zusätzliche Mischung durch ein Mischsystem erfolgen. Vorteilhaft wird der Mischbehälter evakuiert.

Mit dem Mischverfahren (Variante B) können dieselben Schritte wie bei Mischverfahren (Variante A) durchgeführt werden. Besonders effizient kann damit der Schritt c) des Einmischens des Stärkepulvers in die Vormischung 2 durchgeführt werden.

Dieses Mischverfahren ist insbesondere vorteilhaft im Hinblick auf die Zeit, die zur Herstellung der Giessmasse 11 benötigt wird. Ausserdem kann sehr schnell eine sehr hohe Homogenität der Mischung erreicht werden. Beispiele für solche Mischsysteme, die für den Mischkreislauf zur Einmischung und Homogenisierung von Pulvern eingesetzt werden können, sind der FRISTAM PM/PMV oder die YTRON-ZC Pulverlöseanlage.

### Kurze Mischzeit

Während die Herstellung einer Gelatine-Giessmasse 11 typischerweise einige Stunden dauert und die Herstellung von alternativen vegetarischen Giessmassen 11 aufwendig ist und im Bereich von 2 h oder länger dauert, kann die Giessmasse 11 gemäss Mischverfahren Variante A oder Mischverfahren Variante B vergleichsweise sehr einfach erfolgen und innerhalb von weniger als 1 h erhalten werden, insbesondere innerhalb von weniger als 45 min, vorteilhaft sogar innerhalb von weniger als 30 min. Mit dem Mischverfahren (Variante B) kann in einer vorteilhaften Ausführung die Mischung innerhalb von weniger als 25 min, vorteilhaft weniger als 20 min erfolgen.

### Transfer in den Vorratstank

Die homogenisierte Giessmasse 11 wird anschliessend in den Vorratstank 2 transferiert, wo die Giessmasse 11 zwischengelagert wird, und von wo die Giessmasse 11 dann, wenn das Rotary-Die-Verfahren beginnt, zu den Verteilkästen 12 gepumpt wird. Vorratstanks 2 sind typischerweise beheizt und werden vorteilhaft auf eine Temperatur geregelt, welcher der vorteilhaften Temperatur der Giessmasse 11 entspricht, welche oben spezifiziert ist.

In einer vorteilhaften Ausführung wird die Giessmasse 11 in den Vorratstank 2 gepumpt, während der Vorratstank 2 auf einem Vakuum gehalten wird, welches in einer vorteilhaften Ausführung einen absoluten Druck von höchstens 80 mbar aufweist, vorteilhaft von höchstens 70 mbar. Dadurch findet beim Einströmen in den Vorratstank 2 eine zusätzliche Evakuierung und Beseitigung von Luftblasen statt. Es ist sogar möglich, dass eine noch deutlich mit Luftblasen versetzte Giessmasse 11 damit noch vollständig von diesen Luftblasen befreit wird.

In einer vorteilhaften Ausführung wird die Giessmasse 11 beim Transfer durch das Vakuum in den Vorratstank 2 eingesogen, ohne dass die Giessmasse 11 gepumpt werden muss.

### Giessen der Giessmasse und Umformung der Giessmasse zu einem Band

Die Giessmasse 11 wird aus einem Verteilkasten 12, die unten einen Spalt aufweist, durch diesen Spalt auf die Giesstrommel 13 gegossen.

Die Verteilkästen 12 müssen nicht zwingend temperiert werden, da sie durch die kontinuierlich durchlaufende Giessmasse 11 temperiert werden.

In einer vorteilhaften Ausführung werden die Verteilkästen 12 aktiv temperiert auf die vorteilhafte Temperatur der Giessmasse 11, wie diese bei der Herstellung der Giessmasse 11 spezifiziert ist.

Die Giessmasse 11 kann drucklos, d.h. bei Atmosphärendruck, gegossen werden oder es wird unter Druck gegossen, womit auch hoch viskose Giessmassen 11 gut vergossen werden können.

Die Verteilkästen 12 werden kontinuierlich mit Giessmasse 11 versorgt, oder in Intervallen, wobei eine Füllstandregelung dafür sorgt, dass der Füllstand in etwa konstant bleibt.

Beim Giessen wird kontinuierlich ein zunächst flüssiger Film 28 auf die Giesstrommel 13 gegossen, wo die Giessmasse 11 durch Temperaturerhöhung zu einem mehr oder weniger festen Band 29 verfestigt wird, an dem die Dicke gemessen wird.

Die Dicke dieses Bandes 29 liegt vorteilhaft im Bereich von 0,5 bis 1,0 mm, noch vorteilhafter im Bereich von 0,55 bis 0,90 mm, und noch vorteilhafter im Bereich von 0,60 bis 0.80 mm.

Die Giesstrommeln 13 werden vorteilhaft auf eine Temperatur, gemessen durch ein **IR-**Thermometer auf der zylindrischen Oberfläche der Giesstrommeln 13, von 90 bis 115 °C geheizt, noch vorteilhafter auf 95 bis 110 °C, noch vorteilhafter auf 97 bis 108 °C, noch vorteilhafter auf 98 bis 107 °C, und noch vorteilhafter auf 99 bis 106 °C.

Die Temperaturverteilung über die Breite des Giesstrommel 13, welche etwas grösser ist als die Breite des Bandes 29, sollte möglichst gleichförmig sein. In einer vorteilhaften Ausführung liegen die Temperaturen in einem Bereich von weniger als 5 °C, noch vorteilhafter von weniger als 3 °C, noch vorteilhafter von weniger als 2 °C, noch vorteilhafter von weniger als 1 °C, und noch vorteilhafter von weniger als 0,7 °C.

Die Giesstrommeln 13 werden vorteilhaft mit einem flüssigen Medium beheizt, das die Giesstrommeln 13 durchfliesst. In einer vorteilhaften Ausführung ist dieses flüssige Medium Wasser.

### E-Modul des Bandes

Die Festigkeit bzw. Steifigkeit des Bandes 29 ist ein wichtiger Faktor für ein robustes Verfahren bei hoher Produktionsgeschwindigkeit. Insbesondere wird dafür eine gute Festigkeit bzw. Steifigkeit benötigt. Zur Charakterisierung ist der E-Modul des Bandes 29 geeignet.

Durch die besonders vorteilhaften Rezepturen der Giessmasse 11, womit auch eine optimale Verschweissung der beiden Kapselhälften erreicht werden konnte, konnte auch bezüglich des E-Moduls des Bandes 29 eine wichtige Verbesserung erhalten werden im Vergleich zum Stand der Technik, wo ein E-Modul von 0,05 MPa als obere Grenze gilt.

In einer vorteilhaften Ausführung weist der E-Modul des Bandes 29 in MPa einen Wert von mehr als 0,50 auf, vorteilhaft von mehr als 0,55, noch vorteilhafter von mehr als 0,60, noch vorteilhafter von mehr als 0,65, noch vorteilhafter von mehr als 0,70, noch vorteilhafter von mehr als 0,75, noch vorteilhafter von mehr als 0,80.

Gegenüber 0,05 MPa bedeutet bereits eine Steigerung von 0,01 MPa eine sehr bedeutende Verbesserung.

### Stärke-Weichkapsel

Die Rezeptur der wasserfreien Hülle der frischen Stärke-Weichkapsel 22 ergibt sich direkt aus der Rezeptur der trockenen Giessmasse 11, aus der das Band 29 entsteht, aus dem wiederum die frische Weichkapselhülle entsteht. Durch Trocknung dieser frischen Weichkapselhülle entsteht schliesslich die fertige Weichkapselhülle, die sich somit nur im Wassergehalt von der Rezeptur der Giessmasse 11 unterscheidet und einen RestWassergehalt im Bereich von 7 bis 12% aufweist.

Die Zusammensetzung der fertigen Weichkapselhülle wird praktischerweise auf die wasserfreie, fertige Weichkapselhülle bezogen.

Die wasserfreie Stärke-Weichkapselhülle weist somit folgende Komponenten auf:
a) 40 - 80 Gew.-% Stärke,
b) 20 - 65 Gew.-% Weichmacher,
d) maximal 7 Gew.-% ein Verdickungsmittel, und
e) gegebenenfalls maximal 10 Gew.-% gelöste Stärke.

Betreffend vorteilhafte Anteile von Stärke, granulärer Stärke, Weichmacheranteile, vorteilhafte Verdickungsmittel und deren vorteilhafte Anteile sowie betreffend vorteilhafte Anteile an gelöster Stärke, gelten die Angaben, die zur Rezeptur der Giessmasse gemacht worden sind.

Für ein robustes Verfahren bei hoher Produktionsgeschwindigkeit und gute Qualität der Produkte ist ein möglichst festes Band von grosser Bedeutung. Alle vegetarischen Weichkapselverfahren sind mit dem Problem konfrontiert, dass die Bänder sehr weich sind, wodurch die Prozesse insbesondere bei der Produktionsgeschwindigkeit limitiert werden und damit auch teuer werden. Ein festeres Band verschafft also einen wichtigen wirtschaftlichen Vorteil. Es wurde gefunden, dass Verdickungsmittel in dieser Hinsicht einen sehr überraschenden Effekt erzeugen, der für ein festes Band sehr vorteilhaft ist.

Die Rezepturen für die erfindungsmässe Weichkapseln weisen ein Verdickungsmittel auf. Die Funktion dieses Verdickungsmittels bestand ursprünglich darin, die Giessmasse zu stabilisieren. In der Giessmasse, die im Wesentlich aus einer Flüssigkeit aus Wasser und Weichmacher besteht sind die Partikel der granulären Stärke suspendiert. Die Dichte dieser teilkristallinen Partikel ist deutlich höher als die Dichte der Flüssigkeit, weshalb die granuläre Stärke sedimentiert, wenn die Giessmasse stehen gelassen wird. Mit dem Verdickungsmittel wurde die Viskosität der Giessmasse stark erhöht, sodass sie beim Stehenlassen homogen bleibt.

Erstaunlicherweise wurde gefunden, dass schon geringe Anteile an Verdickungsmittel die Festigkeit des Bandes deutlich positiv beeinflussen.

Der Fachmann würde keinen Einfluss des Verdickungsmittels erwarten, denn das Band besteht ja aus Stärke-Makromolekülen und das Verdickungsmittel besteht ebenfalls aus Makromolekülen. Die Festigkeit des Bandes nimmt mit der Grösse der Makromoleküle zu, wobei hier aber sowohl Stärke Makromoleküle wie auch Verdickungsmittel Makromoleküle sehr gross sind und dann Unterschiede nicht mehr wirklich relevant sind.

Das Verdickungsmittel Xanthan beispielsweise hat ein Molekulargewicht bis 10⁶ g/mol und Stärke bis 10⁷ g/mol. Wenn also für das Verdickungsmittel einen Einfluss auf die Festigkeit erwartet werden sollte, dann wäre dies im Vergleich zu Stärke ein geringer, negativer Einfluss. Allerdings sind die Verdickungsmittel typischerweise recht steife Makromoleküle, was sich auch positiv auf die Festigkeit auswirkt. Insbesondere ist Xanthan ein besonders steifes Makromolekül, wodurch das im Vergleich mit Stärke der Einfluss des kleineren Molekulargewichts auf die Festigkeit etwas kompensiert wird. Insgesamt wird jedoch kein merklicher Einfluss erwartet.

Nun wurde aber sehr überraschend ein sehr ausgeprägter Einfluss gefunden, insbesondere bei Xanthan. Dieser Effekt hängt mit der Mikrostruktur des Bandmateriales zusammen, das aus gelatinisierten Stärke-Partikeln besteht, die dicht aneinander liegen. Diese Struktur ist entstanden, indem die granulären Stärkekörner infolge Temperaturerhöhung gelatinisiert sind und dabei die flüssige Phase von Wasser und Weichmacher, in der sie suspendiert waren, vollständig aufgesaugt haben, womit die Stärkekörner massiv grösser geworden sind, bis keine Flüssigkeit mehr da war, um aufgesaugt zu werden. Diese stark gequollenen Stärkekörner sind weich und deformierbar.

Beim Aufsaugen der flüssigen Phase wurde hingegen das Verdickungsmittel nicht aufgenommen, da die Makromoleküle des Verdickungsmittels zu gross sind, um in die Stärkekörner hineinzudiffundieren. Die Makromoleküle des Verdickungsmittels bleiben also ausserhalb der Stärkekörner und lagern sich an der Oberfläche der gequollenen Stärkepartikel an.

Obwohl die Makromoleküle des Verdickungsmittels in der Gesamtmasse sehr stark verdünnt waren, sind sie nun an der Oberfläche der gequollenen Stärkepartikel aufkonzentriert. Hier behindern sie das Gleiten dieser Stärkekörner aufeinander, weshalb die Festigkeit des Bandes, das solche eine Mikrostruktur aufweist, deutlich zunimmt.

Dieser Effekt der Behinderung des Gleitens der Stärkekörner nimmt mit dem Anteil des Verdickungsmittels zunächst langsam, dann verstärkt zu, bis die Stärkekörner flächendeckend von Verdickungsmittel umhüllt sind. Dann kommt ein neuer Mechanismus hinzu, der zur Folge hat, dass eine weitere Erhöhung des Verdickungsmittels einen noch grösseren Effekt hat, weil eine zusätzliche Synergie ins Spiel kommt.

Bei Beton wird Armierungseisen eingelegt, um die Festigkeit der Betonstrukturen deutlich zu erhöhen. Die Armierungseisen sollen die Betonstrukturen durchgehend durchdringen. Wenn nur Teile einer grösseren Betonstruktur armiert sind und dazwischen nicht armierte Bereiche vorliegen, dann ist die Festigkeit durch die schwächsten Bereiche bestimmt. Es wie bei einer Kette, deren Festigkeit durch das schwächste Glied limitiert wird.

Bei der beschriebenen Mikrostruktur formen die Makromoleküle des Verdickungsmittels eine Art von Aussenarmierung, wenn man mit armiertem Beton vergleicht. Ist die Aussenarmierung der Stärkekörner flächendeckend und in diesem Sinne vollständig, wird dadurch ein besonders hoher Beitrag an die Festigkeit erhalten. Darum sind entsprechende Anteile an Verdickungsmittel besonders vorteilhaft.

Der Effekt ist umso grösser, je steifer die beteiligten Makromoleküle sind. Weil Xanthan besonders steife Makromoleküle aufweist, ist dieses Verdickungsmittel entsprechend besonders vorteilhaft.

Der Effekt wird noch verstärkt, wenn noch eine zusätzliche Synergie genutzt wird, die sich bei tieferem Wassergehalt einstellt, wodurch die Aussenarmierung zusätzlich verstärkt wird.

Die bevorzugten Gehalte an Verdickungsmittel, ergeben sich auf der anderen Seite zu hohen Gehalten hin dadurch, dass die hohen Gehalte die Giessmasse so stark verdicken, dass deren Giessbarkeit zum Problem wird.

Schliesslich kommt noch ein weiterer Effekt. Die Bänder basierend auf Stärke sind klebrig, wodurch das Handling der Bänder wie auch der frischen Kapseln ernsthaft beeinträchtigt wird. Ausserdem wird dadurch auch die Verstreckung der Bänder, die ja möglichst gering sein soll, erhöht, weil es mehr Kraft braucht, die Bänder von der Unterlage abzulösen, auf der sie haften. Die Klebrigkeit wird erstaunlicherweise durch das Verdickungsmittel reduziert und insbesondere dann deutlich reduziert, wenn die Stärkekörner vollständig von Verdickungsmittel ummantelt sind, wo also auch die Aussenarmierung richtig zu wirken beginnt.

Die bevorzugte Zusammensetzung der Weichkapsel kombiniert die verschiedenen Synergien in besonders vorteilhafter Weise.

### Wiedergewinnungsverfahren

Die erfindungsgemässen Weichkapselhüllen umfassen charakteristischerweise Partikel von gelatinisierten Stärkekörner, die dicht aneinander liegend miteinander verbunden sind. Diese gelatinisierten Stärkekörner können im Lichtmikroskop identifiziert und ausserdem auch quantitativ nachgewiesen werden, wodurch ein eindeutiger Nachweis hinsichtlich der für die Weichkapseln verwendete Herstellungs-Technologie möglich ist.

Beispiele solcher Nachweisverfahren werden nachfolgend aufgeführt.

### Wiedergewinnungsverfahren Nr. 1

In einer vorteilhaften Ausführung beträgt der minimale Anteil in Gew.-% der gelatinisierten Stärkekörner in der Weichkapselhülle, der nach Auflösen der Weichkapsel bei 70 °C während 30 min zurückgewonnen werden kann, 30, noch vorteilhafter 40, noch vorteilhafter 50, noch vorteilhafter 55, noch vorteilhafter 60, noch vorteilhafter 65, und noch vorteilhafter 70%.

### Wiedergewinnungsverfahren Nr. 2

In einer weiteren vorteilhaften Ausführung wird der Anteil der gelatinisierten Stärkekörner bestimmt, die nach Auflösen der Weichkapselhülle bei 70 °C während 30 min zurückgewonnen werden kann, auf die Masse des trockenen Films bezogen. Die Bestimmung nach dieser Definition ist einfacher als jene gemäss Wiedergewinnungsverfahren Nr. 1, weil sie auch dann angewendet werden kann, wenn die Zusammensetzung der Weichkapselhülle nicht genau bekannt ist.

Der minimale Anteil in Gew.-% der gelatinisierten Stärkekörner, die zurückgewonnen werden kann, liegt bei 25, noch vorteilhafter bei 35, noch vorteilhafter bei 40, noch vorteilhafter bei 45, und noch vorteilhafter bei 50.

### Beispiele

### Beispiel 1

In Tabelle 1 sind mit den Nummern 1 bis 13 einige Vergleichsrezepturen und die E-Moduli der zugehörigen Bänder gemäss den erwähnten Patentanmeldungen der Anmelderin aufgeführt, und mit den Nummern ab 200500 Rezepturen der vorliegenden Erfindung und die E-Moduli der zugehörigen Bänder.

Die Werte für die E-Moduli zeigen, dass hier eine Verbesserung von gegen 100% erreicht werden konnte. Bänder mit entsprechend hohen E-Moduli können im Rotary-Die-Verfahren einfacher und robuster verarbeitet werden, ermöglichen tiefere Verstreckungsgrade und höhere Produktionsgeschwindigkeiten.

Die Messungen wurden an Filmen von 0,8 mm Dicke durchgeführt, die in der Presse bei 100 °C während 2 min erhitzt wurden.

**Tabelle 1**

| | **Stoffe** | | **Zusammensetzung der trockenen Giessmasse** | | | | | **Wasssergehalt der Giessmasse** | **Eigenschaften** | |
|---|---|---|---|---|---|---|---|---|---|---|
| **Nr.** | **Granuläre Stärke** | **Verdickungsmittel** | **Granuläre Stärke** | **gelöste Stärke** | **SCA** | **Verdickungsmittel** | **Weichmacher** | **H2O-1** | **E-Modul** | **H2O-2** |
| | | | **%** | **%** | **%** | **%** | **%** | **%** | **NPa** | **%** |
| 1 | HP Tapioka | | 62,9 | 3,68 | 0,40 | - | 33,1 | 38,0 | 0,03 | 36,5 |
| 5 | native Tapioca | - | 62,9 | 3,51 | 0,40 | - | 33,1 | 38,0 | 0,03 | 36,8 |
| 9 | native Tapioca | - | 63,6 | 3,37 | - | - | 33,1 | 38,0 | 0,04 | 36,7 |
| 11 | HP Tapioka | Guar | 66,3 | - | - | 0,66 | 33,1 | 38,0 | 0,05 | 37,9 |
| 12 | HP Tapioka | Xanthan | 66,6 | - | - | 0,34 | 33,0 | 38,0 | 0,04 | 37,7 |
| 13 | HP Tapioka | J BKM | 66,3 | - | - | 0,66 | 33,1 | 38,0 | 0,04 | 37,8 |
| 200512 | native Tapioca | Xanthan | 62,3 | - | - | 1,3 | 36,4 | 32,81 | 0,072 | 32,17 |
| 200500 | native Tapioca | Xanthan | 61,7 | - | - | 2,3 | 36,0 | 32,63 | 0,094 | 32,01 |
| 200517 | native Tapioca | Xanthan | 61,9 | - | - | 2,0 | 36,1 | 30,84 | 0,094 | 30,37 |
| HP = Hydroxypropyliert, JBKM = Johannisbrothemmel, SCA = Short Chain Amylose, Weichmacher = Glycerin, H2O-2 = Wassergehalt, der Probe an welcher der E-Modul gemessen wird, ist tyischerweise etwas tiefer als der Wassergehalt des frischen Bandes (H2O-1), da bis zur Messung eine gewisser Wasserverlust durch Trocknung stattfindet. | | | | | | | | | | |

### Beispiel 2

Ein vorteilhaftes Beispiel eines Herstellungsverfahrens von Stärke-Weichkapseln kann mit folgenden Parametern durchgeführt werden:
Giessmasse: 32.6% Wasser. Trockene Giessmasse: 60.3% native Tapioka Stärke, 35.4% Glycerin, 2.20% Siliciumdioxid Ibersil^{®}, 1.98% Verdickungsmittel (vorteilhaft Xanthan), 0.17% Zuckerester.

Als Füllgut wurde beispielhaft Sojaöl eingesetzt, 980 mg pro Kapsel.

Es wurden Formwalzen für die Herstellung von Kapseln des Formats «Oblong 20» verwendet. Durchmesser der Formwalze: 150 mm. Die Drehzahl lag bei 3.5 U/min.

Die Dicke des Bandes betrug 700 µm, und die Verstreckung S = 7%.

Die Aussenbeölung betrug 0.4 g/m².

Die Temperatur des Bandes vor dem Füllkeilt betrug 52 °C. Die Temperatur des Füllkeils betrug 50 °C. Die Formwalzen wurden mit Luft (25 °C) gekühlt. Die Temperatur auf der Aussenseite der Formwalze betrug 37 °C.

Die Kapseln hatten nach der Formwalze eine Aussentemperatur von 35 °C und eine Innentemperatur von 32 °C

Nach der Formung wurden die Kapseln im Förderkanal aussen beölt und mit Luft (22 °C) gekühlt.

Zur Vorkonditionierung wurden die Kapseln in zwei Tumblern in Serie mit Kühlluft (22 °C) gekühlt.

Nach der Vorkonditionierung betrug die Temperatur der Kapseln aussen und innen 23 °C.

Von 4512 produzierten Kapseln wies keine Weichkapsel ein Leck auf (Leckrate 0%).

### Messmethoden

### E-Modul

Die Länge der gesamten Zugprüfprobe ist 35 mm, wobei die Länge zwischen den Einspannklemmen, d.h. die Länge, die gedehnt wird, 20 mm beträgt. Die Breite liegt bei 2 mm und die Dicke bei 0.8 mm. Entsprechende Proben wurden aus einem Stärkefilm ausgestanzt.

Die Zugprüfung wurde auf einer Instron 5542 Zugprüfmaschine durchgeführt. Kraftaufnehmer war eine 50 N Kraftmessdose, die Dehngeschwindigkeit betrug 50 mm/min. Der E-Modul wurde aus der mittleren Steigung des Verlaufs im Kraft-Weg Diagramm erhalten, wobei hierzu der Bereich bis zu 100% Dehnung herangezogen wurde.

### Wiedergewinnungsverfahren

Die Wiedergewinnungsverfahren dienen dazu, die in Figur 3(b) gezeigten, gelatinisierten Stärkekörner quantitativ aus der Weichkapselhülle zu isolieren und somit wiederzugewinnen.

Die Wiedergewinnungsverfahren werden wie folgt durchgeführt: Eine Probemenge von 100 - 150 mg (Trockenmasse M0) der Weichkapselhülle wird bei 70 °C zusammen mit 7 g demineralisiertem Wasser in einem Reagenzglas während 30 min unter langsamem Rühren mit einem Magnetrührer gequollen und/oder gelöst. Danach wird das Reagenzglas zentrifugiert, bis die ungelösten Bestandteile sedimentiert haben und der Überstand klar geworden ist. Der Überstand wird dann dekantiert. Dann werden 7 g demineralisiertes Wasser zugegeben und mit dem Sediment verrührt, sowie erneut zentrifugiert und dann dekantiert. Dieser Vorgang wird nochmals wiederholt, um sicherzustellen, dass sich keine löslichen Bestandteile mehr im Sediment befinden. Dieses Sediment besteht bei einer Weichkapselhülle aus gelatinisierter Stärke und Weichmacher, im Wesentlichen aus gelatinisierten Stärkekörnern und Wasser. Schliesslich wird das Sediment während 48h bei 80 °C über Phosphorpentoxid getrocknet und davon die trockene Masse (M1) bestimmt.

Die Masse M1, die nach dem Lösevorgang zurück gewonnen werden kann, entspricht der in gelatinisierter Form vorliegenden Stärke und ihr Anteil bezogen auf das Trockengewicht der Probe ergibt sich somit in Gew.-% zu 100 × M1/M0. Weichmacher, gelöste Stärke oder Verdickungsmittel werden bei diesem Verfahren nicht zurückgewonnen, da sie in dem Wasser gelöst sind, das dekantiert wird.

Der Anteil der Stärke, die nach dem Lösevorgang zurück gewonnen werden kann, ergibt sich bei einem Weichkapselhülle bestehend aus Stärke und Weichmacher, in Gew.-% zu 100 × M1/(M0 × (1-(WM/100)), wobei WM der Anteil in Gew.-% des Weichmachers der trockenen Mischung ist. Gegebenenfalls weist die Weichkapselhüllte neben den Stärkepartikeln höchstens noch minimale Anteile an unlöslichen Bestandteilen auf wie z.B. Pigmente (typischerweise < 0,5%) oder Füllstoffe wie Titandioxid (typischerweise < 1,5%). Solche Bestandteile werden bei der Trockenmasse M0 und der Masse M1 gegebenenfalls subtrahiert, sie beeinflussen das Resultat der Analyse ansonsten jedoch nur in unwesentlichem Umfang.

### Wassergehalt

Der Wassergehalt wird bestimmt, indem eine Probe im Bereich von 100 bis 500 mg in einem Ofen bei 80 °C über dem Trocknungsmittel Phosphorpentoxid während 24 h getrocknet und der damit verbundene Gewichtsverlust ermittelt wird.

Die vorliegende Erfindung ist in ihrem Umfang nicht auf die hier beschriebenen spezifischen Ausführungsformen beschränkt. Vielmehr ergeben sich für den Fachmann aus der Beschreibung und den dazugehörigen Figuren zusätzlich zu den hier offenbarten Beispielen verschiedene weitere Modifikationen der vorliegenden Erfindung, die ebenfalls in den Schutzbereich der Ansprüche fallen.

Zusätzlich werden in der Beschreibung verschiedene Referenzen zitiert, deren Offenbarungsgehalt hiermit in deren Gesamtheit durch Referenz in die Beschreibung mit aufgenommen wird.

### Bezugszeichenliste

- 1: Mischer zur Herstellung der Giessmasse
- 2: Vorratstank für die Giessmasse
- 3: Vorratsgefäss für das flüssigen Füllgut
- 4: Rotary-Die-Vorrichtung
- 5: Förderkanal
- 6: Vorkonditionierung
- 7: Konditionierung
- 8: Qualitätskontrolle, Sortierung, Verpackung
- 11: Giessmasse
- 12: Verteilkasten
- 13: Giesstrommel
- 14: Polierwalze
- 15: Abzugswalze
- 16: Beölungswalze
- 17: Beölungswalze
- 18: Führungswalze
- 18a: Stützriemen
- 19: Füllgut
- 20: Füllkeil
- 21: Formwalze
- 21a: Form
- 22: Frisch gebildete Weichkapseln
- 23: Netz bestehend aus den zwei aufeinanderliegenden Bändern
- 24: Abstreifrolle
- 25: Richtrolle
- 26: erstes Förderband
- 27: zweites Förderband
- 28: flüssiger Film aus Giessmasse
- 29: gegossenes Band
- R1: Radius der Giesstrommel
- R2: Radius der Formwalze
- V1: Oberflächengeschwindigkeit der Giesstrommel
- V2: Oberflächengeschwindigkeit der Formwalze
- 30: Führungswalze, angetrieben
- 31: Beölungswalze Aussenbeölung, angetrieben
- 32: Beölungswalze Innenbeölung, angetrieben
- 33: Führungswalze, nicht angetrieben
- 34: Führungswalze, nicht angetrieben
- 34a: Stützriemen
- 35: Führungswalze, nicht angetrieben
- 36: Heizvorrichtung/Kühlvorrichtung
- 37: Thermoelement
- 38.1, 38.2: Begleitendes Kühlelement
- 39: Ablöserolle
- 44: Rotary-Die-Verkapselungseinheit
- 45: Giesseinheit
- 51: Rolle
- 52: Kühleinheit
- 53: Kühlband
- 54: Führungsrolle
- V1: Umfanggeschwindigkeit der Giesstrommel
- V2: Umfanggeschwindigkeit der Formwalze
- α: Umlenkwinkel
- K: Kontrolleinheit
- S: Verstreckung (relative Streckung in einer Achse)

## Patentansprüche

1. Giessmasse (11) zur Herstellung von auf Stärke basierenden Weichkapseln, **dadurch gekennzeichnet, dass** die Giessmasse (11) 20 - 45 Gew.-% Wasser aufweist und die trockene Giessmasse folgende Komponenten umfasst:
40-80 Gew.-% Stärke, wobei mehr als 80 Gew.-% der Stärke als Partikel granulärer nativer Stärke vorliegt;
20-65 Gew.-% Weichmacher;
0.5-5 Gew.-% Verdickungsmittel.

2. Giessmasse nach Anspruch 1, wobei die native Stärke native Tapioka-Stärke ist.

3. Giessmasse nach Anspruch 1 oder 2, wobei das Verdickungsmittel mindestens eine Komponente aus folgender Liste enthält: Guarkernmehl, Johannisbrotkernmehl, Xanthan; oder wobei das Verdickungsmittel im wesentlichen Xanthan ist.

4. Giessmasse nach einem der Ansprüche 1 bis 3, wobei die die trockene Giessmasse 0.5-4 Gew.-% Verdickungsmittel umfasst, und das Verdickungsmittel im wesentlichen Xanthan ist.

5. Giessmasse nach einem der Ansprüche 1 bis 4, wobei die Giessmasse (11) 20-37 Gew.-% Wasser aufweist.

6. Verfahren zur Herstellung von Weichkapseln mit einem Rotary-Die-Verfahren, **dadurch gekennzeichnet, dass** das Verfahren folgende Verfahrensschritte aufweist:
a) Bereitstellen einer Giessmasse (11), wobei die Giessmasse (11) eine Giessmasse nach einem der Ansprüche 1 bis 5 ist;
b) Giessen der Giessmasse (11) mit einem Verteilkasten (12) auf eine Giesstrommel (13);
c) Umwandlung der Giessmasse (11) auf der Giesstrommel (13) zu einem Band (29) mittels Temperaturerhöhung der Giessmasse (11);
d) Abziehen des Bandes (29) von der Giesstrommel (13) und Zuführen des Bandes (29) zu einer Rotary-Die-Verkapselungseinheit (44) bestehend aus Füllkeil (20) und zwei Formwalzen (21);
e) Verkapselung eines Füllgutes (19) in der Rotary-Die-Verkapselungseinheit (44), wobei die Weichkapseln (22) gebildet werden;
wobei im Schritt d) eine Orientierung der im Band (29) enthaltenen Makromoleküle gering gehalten wird, so dass die Orientierung der Makromoleküle am Ende von Schritt d) in minimalem Umfang vorliegt.

7. Verfahren nach Anspruch 6, wobei in Schritt d) eine Verstreckung (S) des Bandes (29) gering gehalten wird, insbesondere kleiner als 20% ist, wobei die Verstreckung (S) in Prozent aus der Umfanggeschwindigkeit (V1) der Giesstrommel (13) und der Umfanggeschwindigkeit (V2) der Formwalze (21) der Rotary-Die-Verkapselungseinheit (44) mit S = 100*(V2-V1)/V1 erhalten wird.

8. Verfahren nach Anspruch 6 oder 7, wobei auf die Oberfläche des Bandes (29), welche schliesslich die Aussenseite der Hülle der Weichkapsel (22) bildet, in Schritt d) im Verfahrensbereich ab Giesstrommel (13) bis Formwalze (21), über eine Aussenbeölung Öl in einer Menge von höchstens 2 g/m² aufgetragen wird.

9. Verfahren nach einem der Ansprüche 6 bis 8, wobei die Temperatur des Bandes (29) unmittelbar vor dem Einzug unter den Füllkeil (20) der Rotary-Die-Verkapselungseinheit (44) im Bereich von 15 bis 60 °C liegt.

10. Verfahren nach einem der Ansprüche 6 bis 9, wobei der Füllkeil (20) der Rotary-Die-Verkapselungseinheit (44) im Innern eine Temperatur im Bereich von 10 bis 60 °C aufweist.

11. Verfahren nach einem der Ansprüche 6 bis 10, wobei die Formwalzen (21) der Rotary-Die-Verkapselungseinheit (44) auf ihrer Oberfläche eine Temperatur im Bereich von 0 bis 45 °C aufweisen.

12. Verfahren nach einem der Ansprüche 6 bis 11, wobei die Weichkapseln einer Konditionierung (7) zugeführt und dabei getrocknet werden, wobei die Weichkapseln nach der Konditionierung bei Raumtemperatur im Gleichgewicht sind mit einer Luftfeuchtigkeit von 13 bis 40%, vorteilhaft von 15 bis 37%, noch vorteilhafter von 17 bis 35%, und noch vorteilhafter von 19 bis 33%.

13. Verfahren nach einem der Ansprüche 6 bis 12, wobei die Formwalzen (21) der Rotary-Die-Verkapselungseinheit (44) mit einer Umfanggeschwindigkeit (V2) drehen, die bei einem Durchmesser der Formwalzen (21) von 150 mm einer Drehgeschwindigkeit von mehr als 3.5 U/min entspricht, vorteilhaft von mehr als 4 U/min, noch vorteilhafter von mehr als 4.5 U/min, und noch vorteilhafter von mehr als 5 U/min.

14. Stärke-Weichkapsel, hergestellt aus einer Giessmasse nach einem der Ansprüche 1 bis 5; und/oder hergestellt nach einem Verfahren nach einem der Ansprüche 6 bis 13.

15. Stärke-Weichkapsel nach Anspruch 14, wobei mit dem Wiedergewinnungsverfahren Nr. 2 aus der Hülle der Weichkapsel mindestens 25 Gew.-% gelatinisierte Stärkekörner zurückgewonnen werden können, bezogen auf das Gewicht der wasserfreien Weichkapsel-Hülle.
